# EUROPEAN PATENT APPLICATION

(11) **EP 4 218 657 A1**
(43) Date of publication of application: **02.08.2023**
(21) Application number: 21871594.4
(22) Date of filing: 24.09.2021
(51) Int. Cl.: A61B 90/14, A61G 13/12

(54) **SUPPORT UNLOCKING STRUCTURE, JOINT LOCKING MECHANISM AND SURGERY ASSISTING ROBOT SYSTEM**

(30) Priority: 25.09.2020 CN 202022133937 U; 25.09.2020 CN 202022136246 U; 29.09.2020 CN 202022185435 U
(71) Applicant: Wuhan United Imaging Healthcare Surgical Technology Co., Ltd., Wuhan, Hubei 430206 (CN)
(72) Inventor: LI, Sheng, Wuhan, Hubei 430206 (CN)
(74) Representative: Balder IP Law, S.L.
(86) International application number: PCT/CN2021/120298
(87) International publication number: WO 2022/063225

(57) **Abstract**

A support unlocking apparatus (100), comprising a head holder adaptation mechanism (110) and a clamping mechanism (120); the head holder adaptation mechanism (110) comprises an adaptation assembly (112) connected to a head holder mechanism (510) and a mounting plate (111) on which the adaptation assembly (112) is mounted; the clamping mechanism (120) is arranged on a head holder fixing mechanism (200) and clamps the mounting plate (111), the clamping mechanism (120) has an unlocking state and a locking state; when a drag force applied to the head holder adaptation mechanism (110) exceeds a threshold of the clamping mechanism (120), the clamping mechanism (120) is in the unlocking state, and the mounting plate (111) can move relative to the clamping mechanism (120), and when the clamping mechanism (120) is in the locking state, the mounting plate (111) is fixed in the clamping mechanism (120). When an accident occurs, the clamping mechanism (120) automatically unlocks the mounting plate (111) under the effect of a drag force of the head holder mechanism (510), the position of the head holder mechanism (510) is no longer fixed, and the head holder mechanism can sink along with a surgical bed system (500), preventing a patient (600) from being harmed, and achieving the effect of safety protection. Further disclosed are a joint locking mechanism (140), a head holder fixing mechanism (200) and a surgery assisting robot system.

## Description

### CROSS REFERENCE TO RELATED APPLICATIONS

The present application claims priorities to Chinese Patent Application No. 202022133937.X, entitled "Joint Locking Apparatus, head brace connection apparatus and Surgery Assisting Robot System" and filed on September 25, 2020, Chinese Patent Application No. 202022136246.5, entitled "Support Unlocking apparatus and Surgery Assisting Robot System" and filed on September 25, 2020, and Chinese Patent Application No. 202022185435.1, entitled "Head Brace Fixing Apparatus and Surgery Assisting Robot System" and filed on September 29, 2020. The contents of all above applications are incorporated herein by reference in their entirety.

### TECHNICAL FIELD

The present disclosure relates to the field of medical equipment technology, and particularly to a support unlocking apparatus, a joint locking apparatus, a head brace fixing apparatus, and a surgery assisting robot system.

### BACKGROUND

At present, when the surgery assisting robot system operates on the patient's head, the patient's head is usually located on a head brace of a surgical bed. Moreover, in order to ensure accuracy of a surgical operation, the support arm of the surgery assisting robot system needs to be fixedly connected to the head brace on the hospital bed system, so that the head is fixed in the head brace to help the surgery assisting robot precisely identify the position of the head, and then facilitate accuracy of the later surgical operation. However, when risks such as collapse of the surgical bed occur, the patient's body sinks with the surgical bed, but the head does not sink because the support arm and the brace fix the head, which may cause serious damage to the head, and may be life-threatening in severe cases.

### SUMMARY

The present disclosure aims to provide a support unlocking apparatus, a joint locking apparatus, a head brace fixing apparatus, and a surgery assisting robot system, to address the existing problem of injuries to the head caused by the collapse of the surgical bed.

A support unlocking apparatus is provided. The support unlocking apparatus is provided on a head brace fixing apparatus of a surgery assisting robot system and connected to a head brace apparatus of a hospital bed system. The support unlocking apparatus includes a joint locking apparatus and a clamping apparatus. The joint locking apparatus includes an adapter assembly connected to the head brace apparatus and a mounting plate configured to mount the adapter assembly. The clamping apparatus is provided on the head brace fixing apparatus and configured to clamp the mounting plate. The clamping apparatus has an unlocked state and a locked state. The clamping apparatus is placed in the unlocked state and the mounting plate moves with respect to the clamping apparatus when a drag force applied to the joint locking apparatus exceeds a threshold of the clamping apparatus. The mounting plate is fixed in the clamping apparatus when the clamping apparatus is in the locked state.

In an embodiment, the clamping apparatus includes s a connecting plate and a clamping assembly. The connecting plate is mounted on the head brace fixing apparatus, and the clamping assembly is provided on the connecting plate and clamps the mounting plate.

In an embodiment, the clamping assembly includes a fixing bracket, a first elastic member provided in the fixing bracket, and an abutting member provided at an end portion of the first elastic member. The fixing bracket is connected to the connecting plate, and the abutting member abuts against the mounting plate under an elastic force of the first elastic member.

In an embodiment, the clamping assembly further includes an adjustment member movably provided in the fixing bracket and abutting against the first elastic member. The adjustment member is configured to adjust the elastic force of the first elastic member.

In an embodiment, the mounting plate includes a restricting recess engageable with the abutting member, and the abutting member is capable of moving into or out of the restricting recess.

In an embodiment, the clamping assembly includes a plurality of clamping assemblies arranged symmetrically on both sides of the mounting plate, and each side of the mounting plate is provided with at least one clamping assembly.

In an embodiment, the clamping assembly includes a clamping sleeve provided on the connecting plate, a locking member movably provided in the clamping sleeve, and a pressing tab provided at an end portion of the locking member. The pressing tab abuts against the mounting plate, and the locking member is configured to adjust an abutting force between the pressing tab and the mounting plate.

In an embodiment, the clamping apparatus further includes a guide assembly. The mounting plate is connected to the connecting plate through the guide assembly. The mounting plate is movable with respect to the connecting plate through the guide assembly. The guide assembly includes a first guide member and a second guide member. The first guide member is provided on the mounting plate, the second guide member is provided on the connecting plate, and the first guide member slidingly engages with the second guide member.

In an embodiment, the clamping apparatus further includes a friction member provided on the connecting plate and abutting against the mounting plate.

A surgery assisting robot system is provided, including a support frame, a surgical robotic arm arranged with the support frame, a head brace fixing apparatus, and a support unlocking apparatus according to any one of the above technical features. The support unlocking apparatus is provided on the head brace fixing apparatus. The head brace fixing apparatus is configured to support a head brace apparatus through the support unlocking apparatus. The surgical robotic arm is configured to perform a surgical operation. The support frame is configured to control the movements of the surgical robotic arm and the head brace fixing apparatus.

A joint locking apparatus is provided, including a guide sleeve having a first mounting hole and a second mounting hole, axes of which intersect or are located in different planes, a first link coaxially connected to the first mounting hole, a second link rotatably provided within the second mounting hole, and a first locking assembly provided in the second mounting hole and configured to lock the first link and the second link in position with respect to the guide sleeve.

In an embodiment, the guide sleeve includes a guide body and a guide pressing plate connected to the guide body. The second mounting hole is provided in the guide body. The guide pressing plate surrounds and forms the first mounting hole. A preset distance exists between the guide pressing plate and the guide body.

In an embodiment, the guide pressing plate includes a guide curved plate connected to the guide body and a guide fixing plate connected to the guide curved plate. The guide curved plate surrounds and forms the first mounting hole. The guide fixing plate is parallel to a surface of the guide body with the preset distance between the guide fixing plate and the guide body.

In an embodiment, the first locking assembly includes a locking member and a lever assembly connected to the locking member. The locking member is movably provided in the second mounting hole, and the lever assembly is configured to lock or unlock the locking member. The locking member includes a first locking disc, a second locking disc, and an elastic member provided between the first locking disc and the second locking disc. The lever assembly is configured to lock or unlock the first locking disc and the second locking disc.

In an embodiment, the first locking has a first locking portion, the second locking disc has a second locking portion, and the first locking portion is locked to and engaged with the second locking portion. The first locking portion and the second locking portion are tooth-shaped, or the first locking portion fits the second locking portion in a manner that a protrusion fits a groove.

In an embodiment, the lever assembly includes a connecting shaft and a locking lever rotatably connected to the connecting shaft. The connecting shaft extends through the guide pressing plate and the guide body and is connected to the first locking disc and the second locking disc. The locking lever drives the guide pressing plate to abut against the guide body through the connecting shaft, and allows the first locking disc to engage with and lock to the second locking disc.

In an embodiment, an end portion of the locking lever connected to the connecting shaft has a cam shape.

In an embodiment, the lever assembly further includes a stopper configured to sleeve over the connecting shaft and provided on the guide sleeve. The stopper is configured to limit a position of the locking lever when the locking lever is locked.

In an embodiment, the stopper is provided with a restricting gap, and the locking lever is located within the restricting gap when the locking lever is locked.

A head brace connection apparatus is provided, including a plurality of joint locking apparatuses according to any one of the above technical features. The plurality of joint locking apparatuses are connected in series. A first link of one of the joint locking apparatuses is connected to a first link or a second link of an adjacent joint locking apparatus, and/or, a second link of one of the joint locking apparatuses is connected to a second link or a first link of an adjacent joint locking apparatus.

A surgery assisting robot system is provided, including a support frame, a surgical robotic arm arranged with the support frame, and a head brace connection apparatus according to the above claim. The surgical robotic arm is movably arranged with the support frame, and the head brace connection apparatus is arranged with the support frame and is movable with respect to the support frame, such that the head brace connection apparatus connects to and fixes the head brace apparatus.

A head brace fixing apparatus is provided. The head brace fixing apparatus is provided in a surgery assisting robot system and connected to a head brace apparatus of a hospital bed system. The head brace fixing apparatus includes a telescoping assembly telescopically arranged with a support frame of the surgery assisting robot system and connectable to the head brace apparatus, and a second locking assembly configured to lock or unlock the telescoping assembly. The second locking assembly limits a movement of the telescoping assembly when locking the telescoping assembly.

In an embodiment, the telescoping assembly includes a support arm and a linear motion member, and the support arm is movable with respect to the support frame through the linear motion member.

In an embodiment, the linear motion member includes a third guide member and a slide member slidingly engaged with the third guide member. The third guide member is provided in an axial direction of the support arm, and the slide member is arranged with the support frame.

In an embodiment, the second locking assembly includes a transmission member and a braking member. The braking member is arranged with the support frame, and the braking member extends through the transmission member to connect to the support arm.

In an embodiment, the transmission member includes a locking gear and a locking rack engaged with each other. The locking gear is rotatably connected to the braking member, the locking rack is provided in an axial direction of the support arm, and the locking gear locks the locking rack when the braking member locks the locking gear.

In an embodiment, the braking member includes an electromagnetic brake with a friction plate, and the friction plate is in contact with the locking gear when the electromagnetic brake is powered off.

In an embodiment, the head brace fixing apparatus further includes a restricting assembly provided on the support arm and configured to limit a movement of the linear motion member.

In an embodiment, the restricting assembly includes a restricting member provided at an end portion of the support arm and configured to limit a motion stroke of the third guide member.

In an embodiment, the restricting assembly further includes a shock absorber provided on a surface of the restricting member facing the third guide member, and the shock absorber is capable of abutting against the slide member.

A surgery assisting robot system is provided, including a support frame, a surgical robotic arm arranged with the support frame, and a head brace fixing apparatus according to any one of the above technical features. The surgical robotic arm is movably arranged with the support frame, and the head brace fixing apparatus is arranged with the support frame and is telescopic with respect to the support frame. The head brace fixing apparatus, when extending out of the support frame, is connected to and fixes the head brace apparatus.

By using the above technical solution, the present disclosure at least has the following technical advantages.

With the support unlocking apparatus and the surgery assisting robot system of the present disclosure, during a surgical operation, the support arm drives the support unlocking apparatus to move to the hospital bed system, so that the head brace adapter apparatus engages with the head brace apparatus, and then the clamping apparatus clamps the mounting plate of the head brace adapter apparatus to allow the support arm to support and fix the head brace apparatus. When accidents such as collapse occur during the surgical operation, the sinking of the hospital bed system may apply a downward drag force on the head brace apparatus, and the drag force exceeds the threshold of the clamping apparatus, so that the clamping apparatus is passively unlocked and is in the unlocked state. At this point, the head brace apparatus moves with the hospital bed system with respect to the clamping apparatus through the mounting plate. By locking and unlocking the head brace adapter apparatus with the clamping apparatus, the problem of injuries to the head caused by the collapse of the surgical bed is effectively solved, so that when an accident occurs, the clamping apparatus automatically unlocks the mounting plate under the drag force of the head brace apparatus. As such, the head brace apparatus is no longer fixed in position, and the head brace apparatus can sink together with the hospital bed system, preventing the patient from being injured and providing safety protection.

With the head brace fixing apparatus and the surgery assisting robot system of the present disclosure, during a surgical operation, the telescoping assembly extends out of the support frame of the surgery assisting robot system and moves to the hospital bed system, so that the telescoping assembly is engaged with and locked to the head brace apparatus, and then the locking assembly locks the telescoping assembly to limit the telescoping assembly. The head brace adapter apparatus is locked to the telescoping assembly. The telescoping assembly is locked by the locking assembly to reliably fix the head brace apparatus, which effectively solves the existing problem that the movement of the head of the patient affects positioning accuracy. The movement of the head brace apparatus is limited, and then the head of the patient is also reliably fixed in the head brace apparatus to ensure positioning accuracy and surgical precision, ensure good surgical results, reduce surgical risks caused by head movements, and ensure surgical safety.

According to the joint locking apparatus, the head brace connection apparatus, and the surgery assisting robot system of the present disclosure, the first link is mounted in the first mounting hole of the guide sleeve, the locking assembly is mounted in the second mounting hole of the guide sleeve, and the locking assembly is mounted in the second mounting hole. When locked by the locking assembly, the first link and the second link are fixed in position with respect to the guide sleeve, and the joint locking apparatus is locked. When the locking assembly unlocks the first link and the second link, the first link and the second link can move with respect to the guide sleeve, and the joint locking apparatus is unlocked. The locking and unlocking of the first link and the second link can be provided through the cooperation of the locking assembly and the guide sleeve, which effectively solves the problem that the head brace of the surgical bed cannot be connected to the surgery assisting robot system, and provides the reliable connection between the head brace apparatus and the surgical navigation equipment, so that the head of the patient and the surgery assisting robot system are fixed in position, thereby ensuring the precision of a surgical operation and improving the accuracy of the surgical operation.

### BRIEF DESCRIPTION OF THE DRAWINGS

The accompanying drawings constituting a part of the present disclosure are utilized to provide a further understanding of the present disclosure, and the exemplary embodiments and descriptions of the present disclosure are utilized to explain the present disclosure, and do not constitute improper limitations to the present disclosure. In the drawings:
FIG. 1 is a schematic diagram illustrating a surgery assisting robot system supporting a patient according to an embodiment of the present disclosure;
FIG. 2 is a perspective view of a support unlocking apparatus in the surgery assisting robot system shown in FIG. 1;
FIG. 3 is a sectional view of a clamping assembly of the support unlocking apparatus shown in FIG. 2 according to an embodiment of the present disclosure;
FIG. 4 is a perspective view of the support unlocking apparatus shown in FIG. 2 according to another embodiment of the present disclosure;
FIG. 5 is a perspective view of the support unlocking apparatus shown in FIG. 4, in which no head brace adaptor apparatus is shown;
FIG. 6 is a schematic diagram of the surgery assisting robot system shown in FIG. 1 with the head brace fixing apparatus extending out of a support frame;
FIG. 7 is a schematic diagram of the surgery assisting robot system shown in FIG. 1 with the head brace fixing apparatus retracting into the support frame;
FIG. 8 is a perspective view of a head brace fixing apparatus in a surgery assisting robot system;
FIG. 9 is a partially enlarged view of the head brace fixing apparatus shown in FIG. 6;
FIG. 10 is a sectional view of a connection between a braking member and a locking gear in the head brace fixing apparatus shown in FIG. 8;
FIG. 11 is a perspective view of a head brace connection apparatus in the surgery assisting robot system shown in FIG. 1;
FIG. 12 is a schematic exploded view of a joint locking apparatus in the head brace connection apparatus shown in FIG. 11;
FIG. 13 is a sectional view of the joint locking apparatus shown in FIG. 11 in a locked position; and
FIG. 14 is a sectional view of the joint locking apparatus shown in FIG. 11 in an unlocked position.

100, support unlocking apparatus (head brace connection apparatus); 110, head brace adapter apparatus; 111, mounting plate; 112, adapter assembly; 120, clamping apparatus; 121, connecting plate; 122, clamping assembly; 1221, fixing bracket; 1222, abutting member; 1223, first elastic member; 1224, adjustment member; 1225, clamping sleeve; 1226, pressing tab; 1227, locking member; 123, guide assembly; 1231, first guide member; 1232, second guide member; 140, joint locking apparatus; 141, guide sleeve; 1411, first mounting hole; 1412, second mounting hole; 1413, guide body; 1414, guide pressing plate; 14141, guide curved plate; 14142, guide fixing plate; 142, first link; 1421, connector; 143, first locking assembly; 1431, locking member; 14311, first locking disc; 14312, second locking disc; 14313, second elastic member; 1432, lever assembly; 14321, connecting shaft; 14322, locking lever; 14323, stopper; 143231, restricting gap; 14324, nut member; 144, second link; 1441, connecting disc; 200, head brace fixing apparatus; 210, telescoping assembly; 211, support arm; 212, linear motion member; 2121, third guide member; 2122, slide member; 220, second locking assembly; 221, transmission member; 2211, locking gear; 2212, locking rack; 222, braking member; 2221, output shaft; 230, restricting assembly; 231, restricting member; 232, shock absorber; 300, support frame; 400, surgery mechanical arm; 500, hospital bed system; 510, head brace apparatus; 520, surgical bed; 600, patient; 610, head.

### DETAILED DESCRIPTION

In order to make the purpose, features, and advantages of the present disclosure clearer, the specific implementation modes of the present disclosure will be described in further detail in conjunction with the accompanying drawings. In the following description, numerous specific details are set forth in order to provide a thorough understanding of the present disclosure. However, the present disclosure can be implemented in many other ways different from those described here, and those skilled in the art can make similar improvements without departing from the concept of the present disclosure, so that the present disclosure is not limited by the specific embodiments disclosed below.

In the description of the present disclosure, it should be appreciated that orientations or positional relationships indicated by the terms "center", "longitudinal", "transverse", "length", "width", "thickness", "upper", "lower", "front", "rear", "left", "right", "vertical", "horizontal", "top", "bottom", "inside", "outside", "clockwise", "counterclockwise", "axial", "radial", "circumferential", etc., are based on the orientations or positional relationships shown in the drawings, and are merely for the convenience of describing the disclosure and simplifying the description, rather than indicating or implying that the referred device or element definitely has a certain orientation, or is constructed and operated in a certain orientation, and thus should not be construed as limiting the present disclosure.

In addition, the terms "first" and "second" are merely used for descriptive purposes, and cannot be interpreted as indicating or implying relative importance or implicitly specifying the quantity of indicated technical features. Thus, the feature defined as "first" or "second" may explicitly or implicitly include at least one of the features. In the description of the present disclosure, "plurality" means at least two, such as two, three, etc., unless otherwise specifically defined.

In the present disclosure, unless otherwise clearly specified and limited, terms such as "installation", "coupling", "connection" and "fixation" etc., should be interpreted in a broad sense, for example, which can be a fixed connection or a detachable connection, or formed in one piece; which may be a mechanical connection or an electrical connection; which may be a direct connection or an indirect connection through an intermediate element; which may be an internal communication of two elements or an interaction relationship between two elements, unless otherwise specified limit. Those of ordinary skill in the art can understand the specific meanings of the above terms in the present disclosure according to specific situations.

In the present disclosure, unless otherwise clearly specified and limited, a first feature is "on" or "under" a second feature may mean that the first feature is directly in contact with the second feature, or that the first feature is indirectly in contact with the second feature through an intermediary. Moreover, the first feature is "above", "on" and "upon" the second feature may mean that the first feature is directly above or obliquely above the second feature, or may simply mean that the first feature is higher than the second feature in horizontal height. The first feature is "below", "under" and "beneath" the second feature may mean that the first feature is directly below or obliquely below the second feature, or may simply mean that the first feature is lower than the second feature in horizontal height.

It should be noted that when an element is referred to as being "fixed on" or "provided on" another element, it may be directly on another element or there may be an intermediate element. When an element is referred to as being "connected to" another element, it can be directly connected to another element or there may be an intermediate element. Terms "vertical", "horizontal", "upper", "lower", "left", "right" and other similar expressions used in the disclosure are merely for the purpose of illustration and are not intended to represent the unique implementation mode.

### Embodiment I

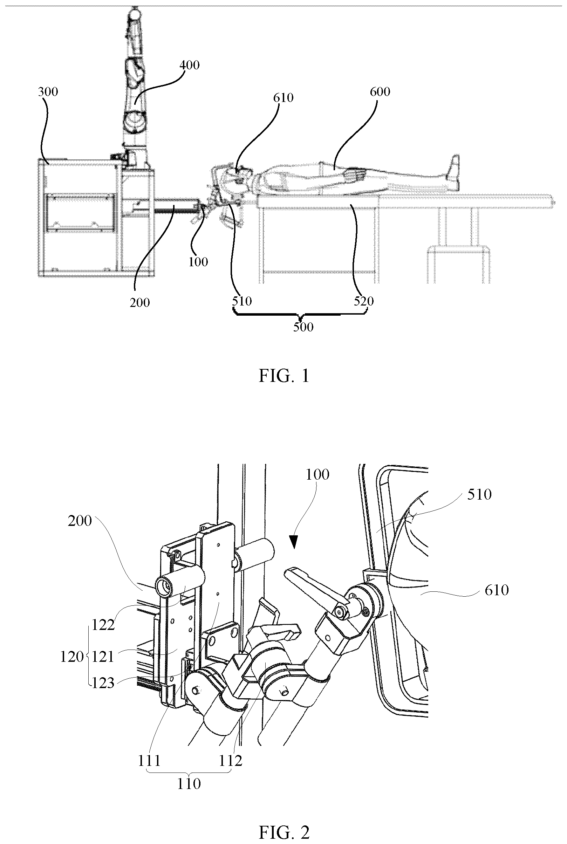

Referring to FIGS. 1-2, the present disclosure provides a support unlocking apparatus 100. The support unlocking apparatus 100 is provided on a head brace fixing apparatus 200 of a surgery assisting robot system and is connected to a head brace apparatus 510 of a hospital bed system 500. In an embodiment of the present disclosure, the support unlocking apparatus 100 is configured to support the head brace apparatus 510. It should be understood that, in other embodiments of the present disclosure, the support unlocking apparatus 100 can also be configured to lock and unlock other components needing to be supported. The support unlocking apparatus 100 of the present disclosure is merely illustrated with the example where the support unlocking apparatus 100 is used with the head brace apparatus 510, and the cooperation between the support unlocking apparatus 100 and other components is substantially the same as that between the support unlocking apparatus 100 and the head brace apparatus 510, which will not be repeated herein. On the one hand, the support unlocking apparatus 100 can reliably support the head brace apparatus 510 to ensure surgery accuracy. On the other hand, when a surgical bed 520 has an accident, such as collapse, the support unlocking apparatus 100 automatically unlocks the head brace apparatus 510, allowing the head brace apparatus 510 to move synchronously with the bed system 500, which provides safety protection prevents a patient 600 from being hurt.

It should be appreciated that the surgery assisting robot system is used with the hospital bed system 500. The hospital bed system 500 includes a surgical bed 520 and a head brace apparatus 510 provided at one end of the surgical bed 520. The patient 600 lies on the surgical bed 520, and the head 610 of the patient is placed on and supported by the head brace apparatus 510. Further, the head brace apparatus 510 includes a head brace and a support rod supporting the head brace on the surgical bed 520. The support rod functions to support, and the head brace is reliably supported on the surgical bed 520 through the support rod. Moreover, the head brace can be raised or lowered with the surgical bed 520 through the support rod. When the surgical bed 520 collapses, the head brace also sinks together with the surgical bed 520.

If the head brace fixing apparatus 200 is directly connected to the head brace apparatus 510, and when the surgical bed 520 for example, collapses, the patient's head 610 is dragged by the sinking body due to the collapse of the hospital bed system 500 and the support of the head brace apparatus 510, and the head brace fixing apparatus 200 pulls the head brace, causing injury to the patient 600. Therefore, in the present disclosure, the support unlocking apparatus 100 is added between the head brace fixing apparatus 200 and the head brace apparatus 510. The head 1421 brace fixing apparatus is connected to the head brace apparatus 510 through the support unlocking apparatus 100, thereby providing locking and unlocking between the head brace fixing apparatus 200 and the head brace apparatus 510. Specifically, when the surgery assisting robot system performs an operation on the patient 600, the head brace fixing apparatus 200 of the surgery assisting robot system moves the support unlocking apparatus 100, and the support unlocking apparatus 100 is connected to the head brace apparatus 510. The head brace apparatus 510 is reliably supported by the head brace fixing apparatus 200, and the surgery accuracy of the surgery assisting robot system during the surgery can be guaranteed. When the surgery table collapses, for example, the surgical bed 520 drags the head brace apparatus 510 downward so that the head brace apparatus 510 is subjected to a drag force. At the same time, the drag force can be applied on the support unlocking apparatus 100, so that the support unlocking apparatus 100 automatically performs the unlocking under the drag force. As such, the support unlocking apparatus 100 no longer locks the head brace apparatus 510, and moves together with the head brace apparatus 510. The head brace apparatus 510 drives the support unlocking apparatus 100 to sink together with the surgical bed 520, thereby avoiding injury to the patient 600 that would otherwise be caused by the head brace fixing apparatus 200 dragging the head brace.

Referring to FIGS. 1-2, in an embodiment, the support unlocking apparatus 100 includes a head brace adapter apparatus 110 and a clamping apparatus 120. The head brace adapter apparatus 110 includes an adapter assembly 112 connected to the head brace apparatus 510 and a mounting plate 111 configured to mount the adapter assembly 112. The clamping apparatus 120 is provided on the head brace fixing apparatus 200 and clamps the mounting plate 111. The clamping apparatus 120 has an unlocked state and a locked state. When the drag force applied to the head brace adapter apparatus 110 exceeds a threshold of the clamping apparatus 120, the clamping apparatus 120 is in the unlocked state, and the mounting plate 111 can move with respect to the clamping apparatus 120. When he clamping apparatus 120 is in the locked state, the mounting plate 111 is fixed in the clamping apparatus 120.

The head brace adapter apparatus 110 is detachably connected to the head brace apparatus 510, which is convenient to use. Specifically, when the surgery assisting robot system performs a surgical operation, the head brace adapter apparatus 110 is connected to the head brace apparatus 510 on the hospital bed system 500, and the head brace adapter apparatus 110 is disconnected from the head brace apparatus 510 after use. In addition, the head brace adapter apparatus 110 and the clamping apparatus 120 are both provided on the head brace fixing apparatus 200. In use, the head brace fixing apparatus 200 moves the head brace adapter apparatus 110 and the clamping apparatus 120 to the surgical bed 520. After the head brace adapter apparatus 110 is connected to the head brace apparatus 510, the head brace fixing apparatus 200 supports the head brace apparatus 510 through the head brace apparatus 510, and thus supports the head 610 of the patient 600, so that the position of the head 610 of the patient 600 is relatively fixed, making it easier for the surgery assisting robot system to obtain the position of the head 610 of the patient 600 to ensure the precision of the surgical operation. After use, the head brace fixing apparatus 200 moves the head brace adapter apparatus 110 and the clamping apparatus 120 to disconnect from the head brace apparatus 510.

The clamping apparatus 120 is a component that performs locking and unlocking of the support unlocking apparatus 100. A locking connection relationship exists between the clamping apparatus 120 and the head brace adapter apparatus 110. During normal use, the clamping apparatus 120 clamps the head brace adapter apparatus 110 to fix the head brace adapter apparatus 110 in position, thereby ensuring that the head brace apparatus 510 precisely fixes the head 610 of the patient 600. When the head brace apparatus 510 is subjected to an external force that exceeds the threshold of the clamping force of the clamping apparatus 120, the clamping apparatus 120 cannot reliably clamp the head brace adapter apparatus 110. At this point, the head brace apparatus 510 moves the head brace adapter apparatus 110 to slide with respect to the clamping apparatus 120.

Specifically, the head brace adapter apparatus 110 includes an adapter assembly 112 and a mounting plate 111. One end of the adapter assembly 112 is fixed on the mounting plate 111, the other end of the adapter assembly 112 is detachably connected to the head brace apparatus 510, and the head brace apparatus 510 is supported by the adapter assembly 112. The mounting plate 111 is connected to the clamping apparatus 120, and the clamping apparatus 120 can clamp the mounting plate 111 to ensure that the position of the adapter assembly 112 is fixed, thereby ensuring that the head brace apparatus 510 reliably supports the head 610 of the patient 600 and ensuring the precision of the surgical operation.

It can be appreciated that the clamping apparatus 120 applies a certain clamping force on the mounting plate 111 to reliably fix the mounting plate 111. If the head brace apparatus 510 is subjected to an external force not exceeding the threshold of the clamping force of the clamping apparatus 120, the clamping apparatus 120 can reliably clamp the mounting plate 111, thereby preventing the head brace adapter apparatus 110 from moving out of position, and reliably fixing the head brace apparatus 510. However, once the external force applied to the head brace apparatus 510 exceeds the threshold of the clamping force of the clamping apparatus 120, the clamping apparatus 120 cannot clamp the mounting plate 111, and the mounting plate 111 may move with respect to the clamping apparatus 120 under the external force. Moreover, the head brace apparatus 510 and the adapter assembly 112 also move synchronously with respect to the clamping apparatus 120.

In other words, the clamping apparatus 120 is kept in the state of clamping the mounting plate 111, i.e., a locked state, so that the mounting plate 111 and the head brace on the mounting plate 111 are fixed. The clamping apparatus 120 is passively unlocked under the external force exceeding the threshold of the clamping force, such that the clamping apparatus 120 enters the unlocked state, and the mounting plate 111 can move with respect to the clamping apparatus 120. When the external force disappears, the clamping apparatus 120 can clamp the mounting plate 111 again.

Exemplarily, when the surgical bed 520 collapses, the surgical bed 520 may generate a sinking force, and the sinking force drags the head brace apparatus 510, so that the head brace apparatus 510 is subjected to a drag force, and the drag force is transferred to the clamping apparatus 120 through the adapter assembly 112 and the mounting plate 111. Since the sinking of the surgical bed 520 creates a larger drag force on the head brace apparatus 510 that exceeds the threshold of the clamping force of the clamping apparatus 120, the clamping apparatus 120 cannot clamp the mounting plate 111. At this point, the mounting plate 111 sinks synchronously with the surgical bed 520 with respect to the clamping apparatus 120 under the drag force, so that the head 610 of the patient 600 can sink together with the surgical bed 520, thereby avoiding dragging the head 610 of the patient 600. It should be understood that, when the head brace apparatus 510 receives an impact force, for example, the clamping apparatus 120 may also be automatically unlocked to ensure safe use.

By the support unlocking apparatus 100 of the above embodiment, the connection between the head brace fixing apparatus 200 and the head brace apparatus 510 can be established, and the locking and unlocking of the head brace adapter apparatus 110 can be provided by the clamping apparatus 120, which effectively addresses the problem of injuries to the head 610 that would be caused by the collapse of the surgical bed 520, and by which the clamping apparatus 120 can automatically unlock the mounting plate 111 under the drag force of the head brace apparatus 510 when an accident occurs, so that the position of the head brace apparatus 510 is no longer fixed and the head brace apparatus 510 can sink with the hospital bed system 500 to prevent the patient 600 from being hurt, which provides safety protection.

Optionally, the adapter assembly 112 includes a plurality of interconnected links and a connecting block arranged at an end of the interconnected links. The connecting block has end face teeth and can be engaged with and connected to a fixing block of the head brace apparatus 510 through a threaded member, with the end face teeth of the connecting block engaging with end face teeth of the fixing block to limit the relative movement of the connecting block, thereby ensuring that the head brace adapting apparatus 110 can reliably support the head brace apparatus 510. Moreover, the movement of the connecting block can be manually driven by medical staff, or can also be driven by rotation of a rotating power source. It is to be noted that the improvement of the present disclosure lies in how to provide the locking and unlocking between the head brace adapter apparatus 110 and the head brace fixing apparatus 200, and the head brace adapter apparatus 110 is an existing structure, the details of which will not be repeated here.

In an embodiment, the clamping apparatus 120 includes a connecting plate 121 and a clamping assembly 122. The connecting plate 121 is mounted on the head brace fixing apparatus 200, and the clamping assembly 122 is provided on the connecting plate 121 and clamps the mounting plate 111. The connecting plate 121 is in a shape of a flat plate and is configured to carry and mount the clamping assembly 122 and the head brace adapter apparatus 110 fixed by the clamping assembly 122. A surface of the connecting plate 121 is mounted on the head brace fixing apparatus 200, and the clamping assembly 122 is mounted on the other surface. The mounting plate 111 is clamped tightly in the clamping assembly 122 so that the mounting plate 111 is reliably fixed in the clamping assembly 122. Optionally, the clamping assembly 122 can directly clamp the mounting plate 111. It should be understood that in other embodiments of the present disclosure, the clamping assembly 122 can also indirectly clamp the mounting plate 111, which will be described in detail later.

The clamping assembly 122 provides a clamping force to tightly clamp the mounting plate 111, and the clamping assembly 122 is in the locked state. When the drag force applied to the head brace adapter apparatus 110 exceeds the threshold of the clamping force of the clamping assembly 122, the clamping assembly 122 is in the unlocked state, and the mounting plate 111 can move with respect to the clamping assembly 122. In other words, the clamping assembly 122 keeps clamping the mounting plate 111, and when the drag force applied to the mounting plate 111 exceeds the clamping force of the clamping assembly 122, the clamping assembly 122 is unlocked. When the drag force applied to the mounting plate 111 is less than the clamping force of the clamping assembly 122 or the clamping assembly 122 is not subjected to any external force, the clamping assembly 122 keeps clamping the mounting plate 111 to lock the mounting plate 111.

Specifically, when the head brace adapter apparatus 110 is normally connected to the head brace apparatus 510 and normally operates, the clamping force of the clamping assembly 122 can reliably clamp the mounting plate 111 to prevent the mounting plate 111 from moving, by which the head brace apparatus 510 is reliably supported and the surgical accuracy is ensured. When the head brace apparatus 510 or the adapter assembly 112 is subjected to a smaller drag force, the drag force is transferred to the clamping assembly 122 through the mounting plate 111. Since the drag force is less than the clamping force of the clamping assembly 122, the clamping assembly 122 can still reliably clamp the mounting plate 111. When the head brace apparatus 510 or the adapter assembly 112 is subjected to a larger drag force, the drag force is transferred to the clamping assembly 122 through the mounting plate 111. Since the drag force exceeds the threshold of the clamping force of the clamping assembly 122, the clamping assembly 122 cannot reliably clamp the mounting plate 111, and the mounting plate 111 can move with respect to the clamping assembly 122 under the drag force.

Referring to FIGS. 2-3, in an embodiment, the clamping assembly 122 includes a fixing bracket 1221, a first elastic member 1223 provided in the fixing bracket 1221, and an abutting member 1222 provided at an end of the first elastic member 1223. The fixing bracket 1221 is connected to the connecting plate 121, and the abutting member 1222 can abut against the mounting plate 111 under an elastic force of the first elastic member 1223. The fixing bracket 1221 has a mounting hole. The first elastic member 1223 is mounted in the mounting hole. One end of the first elastic member 1223 abuts against an inner wall of the mounting hole, and the other end of the first elastic member 1223 abuts against the abutting member 1222. The first elastic member 1223 keeps in a compressed state. The extended elastic force of the first elastic member 1223 pushes the abutting member 1222 to move toward the outside of the mounting hole, so that the abutting member 1222 always abuts against the side wall of the mounting plate 111, which restricts the movement of the mounting plate 111 and thus locks the mounting plate 111. Optionally, the abutting member 1222 is spherical, cylindrical, or has other shapes, as long as it can abut against the mounting plate 111 and limit the position of the mounting plate 111. Optionally, the first elastic member 1223 is a spring.

If the drag force applied to the mounting plate 111 is greater than the threshold of the elastic force of the first elastic member 1223, i.e., the drag force applied to the mounting plate 111 is greater than the abutting force applied to the mounting plate 111 by the abutting member 1222, the mounting plate 111 can slide with respect to the abutting member 1222. If the drag force applied to the mounting plate 111 is less than the threshold of the elastic force of the first elastic member 1223, that is, the drag force applied to the mounting plate 111 is less than the abutting force applied to the mounting plate 111 by the abutting member 1222, or the mounting plate 111 is not subjected to any drag force, the mounting plate 111 is fixed by the abutting member 1222and is thus locked.

In an embodiment, the clamping assembly 122 further includes an adjustment member 1224, which is movably provided in the fixing bracket 1221 and abuts against the first elastic member 1223. The adjustment member 1224 is configured to adjust the elastic force of the first elastic member 1223. The adjustment member 1224 is mounted in the mounting hole and located at an end of the first elastic member 1223 away from the abutting member 1222. Since the abutting member 1222 keeps abutting against the mounting plate 111, the position of the abutting member 1222 remains unchanged. When the adjustment member 1224 moves toward the abutting member 1222 in an axial direction of the mounting hole, the adjustment member 1224 can compress the first elastic member 1223, such that the first elastic member 1223 store energy, thereby increasing the threshold of the elastic force of the first elastic member 1223. The head brace apparatus 510 can be thus fixed in position under a larger drag force. When moving toward the direction away from the abutting member 1222 in the axial direction of the mounting hole, the adjustment member 1224 allows the first elastic member 1223 to extend, so that the first elastic member 1223 releases the energy to decrease the threshold of the elastic force of the first elastic member 1223, thereby improving the sensitivity.

The threshold of the elastic force of the first elastic member 1223 is adjusted by the adjustment member 1224 to meet the requirements of different drag forces. Moreover, requirements for different situations can be satisfied, and safety during a surgical operation can be ensured. Optionally, the inner wall of the mounting hole has an internal thread, and the adjustment member 1224 is a threaded member which is threaded with the internal thread in the mounting hole. When the adjustment member 1224 rotates, the adjustment member 1224 can move along a circumference direction of the mounting hole through the inner thread. It should be understood that in other embodiments of the present disclosure, the adjustment member 1224 can also be, for example, a telescopic rod that adjusts the elastic force of the first elastic member 1223.

In an embodiment, the fixing bracket 1221 includes a mounting housing and a supporting seat connected to the mounting housing. The mounting hole is located in the mounting housing, and the mounting housing plays a carrying role and is configured to mount the abutting member 1222 and the first elastic member 1223. The supporting seat is configured to fix the fixing bracket 1221 on the connecting plate 121. Optionally, the mounting housing and the supporting seat are formed in one piece to reduce the number of parts and facilitate use, and the reliable connection between the mounting housing and the supporting seat can be ensured to avoid breakage between the mounting housing and the supporting seat in use. Optionally, the mounting housing has a cylindrical shape. It should be understood that, in other embodiments of the present disclosure, the cross-sectional shape of the mounting housing may also be a polygon. Optionally, the mounting hole extends through the mounting housing, facilitating the mounting and use of the adjustment member 1224.

In an embodiment, the mounting plate 111 has a restricting recess matched with the abutting member 1222, and the abutting member 1222 can move into or out of the restricting recess. The restricting recess is configured to fix a connection position between the abutting member 1222 and the mounting plate 111. The end portion of the abutment member 1222 is mounted in the restricting recess. When the mounting plate 111 is subjected to a smaller drag force or no drag force, the abutting force of the abutting member 1222 can tightly abut against the mounting plate 111, so that the position of the mounting plate 111 is fixed. At this point, the abutting member 1222 cannot move out of the restricting recess. When the mounting plate 111 is subjected to a larger drag force, the mounting plate 111 moves downward, and the inner wall of the mounting groove presses against the abutting member 1222, so that the abutting member 1222 moves out of the restricting recess. At this point, the mounting plate 111 can move with respect to the abutting member 1222. Optionally, the restricting recess is a curved recess, which is convenient for the abutting member 1222 to move in and out.

In an embodiment, there are a plurality of clamping assemblies 122which are symmetrically arranged on both sides of the mounting plate 111, and there is at least one clamping assembly 122 on each side. The plurality of clamping assemblies 122 are fixed on the connecting plate 121 through respective fixing brackets 1221, so that the plurality of clamping assemblies 122 are symmetrically arranged on both sides of the mounting plate 111. This ensures that the mounting plate 111 is subjected to uniform forces, and ensures that the mounting plate 111 can be reliably locked. The clamping assemblies 122 clamps the mounting plate 111 through the abutting forces output by the clamping assemblies 122 on both sides of the mounting plate 111, ensuring that the mounting plate 111 is fixed reliably.

Exemplarily, the number of clamping assemblies 122 is two, and the two clamping assemblies 122 are arranged symmetrically on both sides of the mounting plate 111. The mounting plate 111 is clamped tightly by the two mounting plates 111, ensuring that the mounting plate 111 is fixed reliably. Optionally, there may be one clamping assembly 122, and a baffle plate is provided on the other side of the mounting plate 111. The mounting plate 111 is clamped with the cooperation of the clamping assembly 122 and the baffle plate, which also realizes the clamping of the mounting plate 111.

Referring to FIGS. 2, 4, and 5, in an embodiment, the clamping assembly 122 includes a clamping sleeve 1225 provided on the connecting plate 121, a locking member 1227 movably provided within the clamping sleeve 1225, and a pressing tab 1226 provided at an end portion of the locking member 1227. The pressing tab 1226 abuts against the mounting plate 111, and the locking member 1227 is configured to adjust the abutting force between the pressing tab 1226 and the mounting plate 111. The clamping sleeve 1225 is provided on the connecting plate 121. The clamping sleeve 1225 is provided with a through clamping opening. The clamping opening is configured to receive the mounting plate 111, and the mounting plate 111 can move along the clamping opening. The pressing tab 1226 can be in contact with a surface of the mounting plate 111. After the locking member 1227 drives the pressing tab 1226 to approach and abut against the mounting plate 111, one surface of the mounting plate 111 is in contact with the pressing tab 1226, and the other surface of the mounting plate 111 is in contact with an inner wall of the clamping opening. The position of the mounting plate 111 is thus fixed by the pressing force of the pressing tab 1226 and the inner wall of the clamping opening.

If the drag force applied to the mounting plate 111 is greater than the threshold of the pressing force of the pressing tab 1226, i.e., the drag force applied to the mounting plate 111 is greater than the pressing force of the pressing tab 1226 on the mounting plate 111, the mounting plate 111 can move with respect to the clamping opening. If the drag force applied to the mounting plate 111 is less than the threshold of the pressing force of the pressing tab 1226 on the mounting plate 111, that is, the drag force applied to the mounting plate 111 is less than the pressing force of the pressing tab 1226 on the mounting plate 111, or the mounting plate 111 is not subjected to any drag force, the mounting plate 111 is fixed by the pressing tab 1226, and the mounting plate 111 is locked.

Additionally, the locking member 1227 can drive the pressing tab 1226 to move, and adjust the pressing force of the pressing tab 1226 on the mounting plate 111 to meet requirements for different drag forces. Optionally, the clamping sleeve 1225 is provided with a threaded hole, and the locking member 1227 is rotatably mounted in the threaded hole. The tight pressing of the mounting plate 111 and the adjustment of the pressing force are realized by rotating the locking member 1227 to drive the pressing tab 1226 to move. Optionally, the number of pressing tabs 1226 may be two or more, and the two or more pressing tabs 1226 are arranged in the clamping openings on both sides, and clamp the mounting plate 111 on both sides thereof.

Referring to FIGS. 2-5, in an embodiment, the clamping apparatus 120 further includes a guide assembly 123. The mounting plate 111 is connected to the connecting plate 121 through the guide assembly 123, and the guide assembly 123 allows the mounting plate 111 to move with respect to the connecting plate 121. The guide assembly 123 can guide the movement of the mounting plate 111 to prevent a change of the position of the head brace apparatus 510 due to the deflection of the mounting plate 111, thereby ensuring the safety of the head 610 of the patient 600. Moreover, when the mounting plate 111 sinks under the drag force, the mounting plate 111 sinks under the guidance of the guide assembly 123, thereby ensuring the movement trajectory of the mounting plate 111 and reducing the sinking speed of the mounting plate 111 to prevent the mounting plate 111 from sinking too fast and dragging the head brace apparatus 510.

In an embodiment, the guide assembly 123 includes a first guide member 1231 and a second guide member 1232. The first guide member 1231 is provided on the mounting plate 111, and the second guide member 1232 is provided on the connecting plate 121. The first guide member 1231 and the second guide member 1232 slidingly engage with each other. After the mounting plate 111 is subjected to the drag force and the clamping assembly 122 unlocks the mounting plate 111, the mounting plate 111 can move under the drag force, and meanwhile, the mounting plate 111 drives the first guide member 1231 to move along the second guide member 1232. Optionally, the first guide member 1231 is a slide rail, and the second guide member 1232 is a slide block. It should be understood that, in other embodiments of the present disclosure, the first guide member 1231 is a slide block, and the second guide member 1232 is a slide rail.

Optionally, the clamping assembly 122 can directly clamp the mounting plate 111 to lock the mounting plate 111. It should be understood that, in other embodiments of the present disclosure, the mounting plate 111 can also clamp the guide assembly 123, and the position limitation of the mounting plate 111 can be achieved by limiting the position of the guide assembly 123. Moreover, when the mounting plate 111 is subjected to the drag force, the drag force may be transferred to the guide assembly 123 through the mounting plate 111, to passively unlock the clamping assembly 122. More specifically, the clamping assembly 122 can clamp the first guide member 1231.

In an embodiment, the clamping apparatus 120 further includes a friction member provided on the connecting plate 121 and abutting against the mounting plate 111. The friction member can increase the friction force between the mounting plate 111 and the connecting plate 121, and the mounting plate 111 can be locked by the friction force of the friction member to ensure the reliable fixing of the mounting plate 111. Optionally, the friction member may include a friction surface, a friction protrusion providing the friction force, or other components capable of generating a large friction force. Optionally, the friction member can be used separately, through which the mounting plate 111 can be reliably fixed. It should be understood that the friction member can also be used in combination with the clamping assembly 122 to further ensure that the adapter assembly 112 can reliably support the head brace apparatus 510.

In an embodiment, the clamping assembly 122 includes a clamping plier, and a jaw of the clamping plier clamps the mounting plate 111. When the drag force applied to the mounting plate 111 exceeds the threshold of the clamping force of the clamping plier, the mounting plate 111 moves along the clamping plier. It should be understood that, in other embodiments of the present disclosure, the clamping assembly 122 may also be other types of components capable of locking and unlocking the mounting plate 111.

Referring to FIGS. 1-2, the present disclosure further provides a surgery assisting robot system, including a surgery assisting robot, a surgical robotic arm 400 and a head brace fixing apparatus 200 provided on the surgery assisting robot, and the support unlocking apparatus 100 described in the above embodiments. The support unlocking apparatus 100 is provided on the head brace fixing apparatus 200, and the head brace fixing apparatus 200 can support the head brace apparatus 510 through the support unlocking apparatus 100. The surgical robotic arm 400 is configured to perform surgery, and the surgery assisting robot controls the movement of the surgical robotic arm 400 and the head brace fixing apparatus 200. One end of the surgical robotic arm 400 is connected to the surgery assisting robot, and the other end can clamp a surgical instrument. Optionally, the surgical instrument includes but is not limited to a puncture needle, a scalpel, etc., and may also be other types of handheld instruments.

In the surgery assisting robot system using the above-mentioned support unlocking apparatus 100, the head brace fixing apparatus 200 is reliably connected to and locks the head brace apparatus 510 through the support unlocking apparatus 100, ensuring that the position of the head 610 of the patient 600 with respect to the surgical robotic arm 400 will not be changed and ensuring the precision of a surgical operation. When accidents such as the collapse of the surgical bed 520 occur, the drag force applied to the head brace apparatus 510 exceeds the threshold of the clamping force of the clamping apparatus 120, and the clamping apparatus 120 cannot clamp the mounting plate 111. At this point, the mounting plate 111 sinks synchronously with the surgical bed 520 with respect to the clamping apparatus 120 under the drag force, so that the head 610 of the patient 600 can sink together with the surgical bed 520 to avoid dragging the head 610 of the patient 600, which provides safety protection and prevents the patient 600 from being hurt.

In an embodiment, the surgical robotic arm 400 is a serial robotic arm and/or a parallel robotic arm. In other words, the surgical robotic arm 400 may include a plurality of serial robotic arms, and surgical operations are implemented by connecting the plurality of serial robotic arms. The surgical robotic arm 400 may further include a plurality of parallel robotic arms, and surgical operations are implemented by connecting the plurality of parallel robotic arms. It should be understood that the surgical robotic arm 400 may further include at least one serial robotic arm and at least one parallel robotic arm, and surgical operations are implemented through the cooperation of the serial robotic arm and the parallel robotic arm. In this case, the parallel robotic arm is located at the end of the serial robotic arm. It can be appreciated that the serial robotic arm includes a plurality of single arms, and adjacent single arms are rotatably connected. The parallel robotic arm can include a platform such as a Stewart platform.

### Embodiment 11

Referring to FIGS. 1 and 6-10, the present disclosure provides a head brace fixing apparatus 200. The head brace fixing apparatus 200 is provided in the surgery assisting robot system and is connected to the head brace apparatus 510 of the hospital bed system 500. The head brace fixing apparatus 200 can fix the head brace apparatus 510 of the hospital bed system 500, so that the position of the head brace apparatus 510 can be fixed without moving. In an embodiment of the present disclosure, the head brace fixing apparatus 200 is configured to support and fix the head brace apparatus 510. It should be understood that, in other embodiments of the present disclosure, the head brace fixing apparatus 200 can also support and fix other components. Only the example where the head brace fixing apparatus 200 of the present disclosure is used with the head brace apparatus 510is illustrated, and the cooperation between the head brace fixing apparatus 200 and other components is substantially the same as that between the head brace fixing apparatus 200 and the head brace apparatus 510, which will not be repeated here.

The head brace fixing apparatus 200 can reliably support and fix the head brace apparatus 510, such that the head brace apparatus 510 can ensure that the head 610 of the patient 600 on the head brace apparatus 510 is fixed in position without any movement. Accordingly, it is ensured that the surgery assisting robot system is fixed in position with respect to the head 610 of the patient 600, which ensures the precision of a surgical operation.

It can be appreciated that the surgery assisting robot system is used in combination with the hospital bed system 500. The hospital bed system 500 includes a surgical bed 520 and a head brace apparatus 510 provided at one end of the surgical bed 520. The patient 600 lies on the surgical bed 520, and the head 610 is placed on the head brace apparatus 510 which supports the head 610 of the patient 600. Further, the head brace apparatus 510 includes a head brace and a support rod supporting the head brace on the surgical bed 520. The support rod plays the role of supporting, and the head brace is reliably supported on the surgical bed 520 through the support rod.

If the head brace is supported only by the support rod of the head brace apparatus 510, the head brace may shake under the gravity of the head 610 on the head brace, so that the position of the head 610 of the patient 600 is not fixed, which affects the positioning accuracy. Therefore, in the present disclosure, the head brace fixing apparatus 200 is utilized to fix the head brace apparatus 510 to further support the head brace apparatus 510, so that the head brace apparatus 510 is reliably fixed, thereby preventing the head brace apparatus 510 from shaking. At this point, a position relationship between the head 610 of the patient 600 and the surgical robotic arm 400 of the surgery assisting robot system can be calibrated, so that the relative position relationship between the head 610 of the patient 600 and the surgical robotic arm 400 is fixed without any relative movement, thereby ensuring the positioning accuracy.

Moreover, the head brace fixing apparatus 200 of the present disclosure is mounted in a support frame 300 of the surgery assisting robot system. When in use, the head brace fixing apparatus 200 can extend out of the support frame 300 and move to approach the head brace apparatus 510 to connect to the head brace apparatus 510. The connection of the head brace fixing apparatus 200 to the head brace apparatus 510 reliably fixes the head brace apparatus 510, thereby preventing the head brace apparatus 510 from shaking, and ensuring positioning accuracy. When performing the surgery, the surgical robotic arm 400 can perform the surgery on the head 610 of the patient 600 according to the calibrated position, thereby ensuring surgical accuracy, avoiding surgical risks that would be caused by the movement of the head 610 of the patient 600, and ensuring the safety throughout a surgical operation. After use, the head brace fixing apparatus 200 is disconnected from the head brace apparatus 510 and can be retracted into the support frame 300, which reduces the occupied space of the surgery assisting robot system and avoids interference with other components, making it easy to use the head brace fixing apparatus 200.

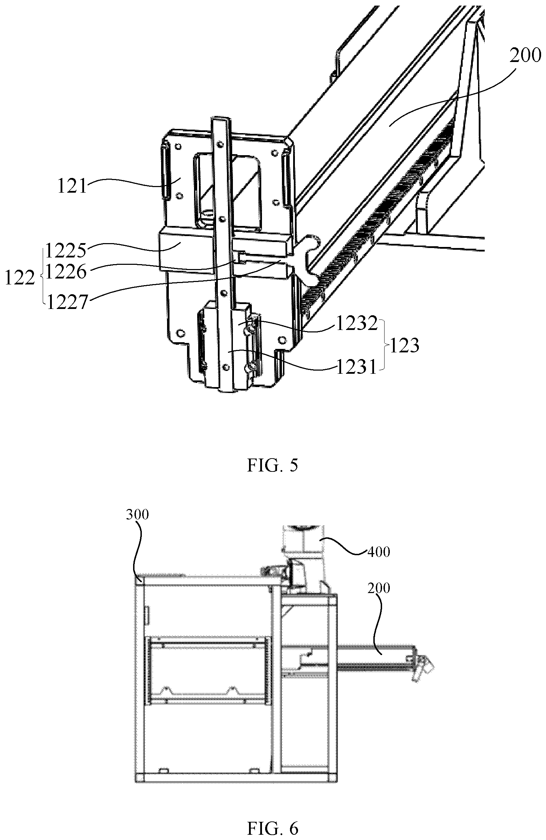

Referring to FIGS. 6-10, in an embodiment, the head brace fixing apparatus 200 includes a telescoping assembly 210 and a second locking assembly 220. The telescoping assembly 210 is telescopically arranged with the support frame 300 of the surgery assisting robot system, and can be connected to the head brace apparatus 510. The second locking assembly 220 is configured to lock or unlock the telescoping assembly 210. When the second locking assembly 220 locks the telescoping assembly 210, the second locking assembly 220 restricts the movement of the telescoping assembly 210.

Optionally, the second locking assembly 220 may be provided on the telescoping assembly 210, and the telescoping assembly 210 is directly locked by the second locking assembly 220. It should be understood that, in other embodiments of the present disclosure, the second locking assembly 220 can also be provided independently of the telescoping assembly 210. When the telescoping assembly 210 needs to be locked, the second locking assembly 220 can be placed on the telescoping assembly 210, and when the telescoping assembly 210 is unlocked, the second locking assembly 220 can be removed from the telescoping assembly 210. In the embodiment, only the case where the second locking assembly 220 is provided on the telescoping assembly 210 is illustrated as an example.

The telescoping assembly 210 is the main portion of the head brace fixing apparatus 200. One end of the telescoping assembly 210 is connected to the support frame 300, and the other end of the telescoping assembly 210 is a connection end detachably connected to the head brace apparatus 510. The telescoping assembly 210 can perform a telescopic movement with respect to the support frame 300. After the telescoping assembly 210 is extended out of the support frame 300, the connection end of the telescoping assembly 210 extends out of the support frame 300 and moves to approach the surgical bed 520. At this point, the connection end of the telescoping assembly 210 can be connected to the head brace apparatus 510. The head brace apparatus 510 is supported by the telescoping assembly 210. After a surgical operation is completed, the telescoping assembly 210 is disconnected from the head brace apparatus 510, and the telescoping assembly 210 can be retracted into the support frame 300, preventing the telescoping assembly 210 from interfering with other components or other surgical operations not needing support.

Since the telescoping assembly 210 is a moving part, it would drive the head brace apparatus 510 to move if it continues to move after connecting to the head 1421 brace apparatus, such that the head brace apparatus 510 cannot precisely fix the head 610 of the patient 600. Therefore, a second locking assembly 220 is involved in the head brace fixing apparatus 200 of the present disclosure, and the telescoping assembly 210 is locked by the second locking assembly 220, so that the telescoping assembly 210 is fixed and cannot perform the telescopic movement. Specifically, when the second locking assembly 220 unlocks the telescoping assembly 210, the telescoping assembly 210 can perform the telescopic movement. When the second locking assembly 220 locks the telescoping assembly 210, the telescoping assembly 210 is fixed and cannot perform the telescopic movement.

When the telescoping assembly 210 is inside the support frame 300, the second locking assembly 220 is in the locked state, and the telescoping assembly 210 is reliably fixed inside the support frame 300 and will not extend out of the support frame 300, which can prevent the telescoping assembly from extending out by a misoperation. When the telescoping assembly 210 needs to extend out, the second locking assembly 220 unlocks it, and the telescoping assembly 210 extends out of the support frame 300 and moves to approach the surgical bed 520. The telescoping assembly 210 is then locked by the second locking assembly 220, such that the telescoping assembly 210 is fixed, and then the telescoping assembly 210 is connected to the head brace apparatus 510. At this point, the telescoping assembly 210 can reliably fix the head brace apparatus 510 to prevent the head brace apparatus 510 from moving and ensure surgical accuracy. After use, the second locking assembly 220 unlocks the telescoping assembly 210 and the telescoping assembly 210 can be retracted into the support frame 300. It should be understood that the telescoping assembly 210 can be first connected to the head brace apparatus 510, and then locked by the second locking assembly 220.

In the head brace fixing apparatus 200 of the above embodiment, the head brace apparatus 510 can be reliably fixed by locking the telescoping assembly 210 with the second locking assembly 220, which effectively solves the problem that the movement of the head 610 of the patient 600 affects the positioning accuracy. The movement of the head brace apparatus 510 is limited, and thus the head 610 of the patient 600 can also be reliably fixed in the head brace apparatus 510, ensuring positioning accuracy, surgical accuracy, and a good surgical result, reducing surgical risks that would be caused by the movement of the head 610, and ensuring the surgery safety.

It should be appreciated that the telescoping assembly 210 can be directly connected to the head brace apparatus 510. At this point, the end portion of the telescoping assembly 210 has a matching portion that is connected to the head brace apparatus 510, and the telescoping assembly 210 is connected to the head brace apparatus 510 by the matching portion. It should be understood that, in other embodiments of the present disclosure, the telescoping assembly 210 can be indirectly connected to the head brace apparatus 510. Optionally, the end portion of the telescoping assembly 210 is detachably connected to a joint locking apparatus 140, and the telescoping assembly 210 is connected to the head brace apparatus 510 through the joint locking apparatus 140 to establish a mechanical connection between the telescoping assembly 210 and the head brace apparatus 510. It should be understood that the telescoping assembly 210 can also be connected to the head brace apparatus 510 through other connecting components.

In the case where the telescoping assembly 210 is connected to the head brace apparatus 510 through the joint locking apparatus 140, the telescoping assembly 210 is locked after the telescoping assembly 210 moves to approach the surgical bed 520. Subsequently, one end of the joint locking apparatus 140 is connected to the connecting end of the telescoping assembly 210, and the other end of the joint locking apparatus 140 is connected to the head brace apparatus 510. It should be understood that the joint locking apparatus 140 can also be connected to the head 1421 brace apparatus first, and then connected to the telescoping assembly 210.

Optionally, the joint locking apparatus 140 includes a plurality of interconnected links and a connecting block arranged at an end of the interconnected links. The connecting block has end face teeth and can be engaged with and connected to a fixing block of the head brace apparatus 510 through a threaded member, with the end face teeth of the connecting block engaging with the end face teeth of the fixing block to limit the relative movement of the connecting block, thereby ensuring that the joint locking apparatus 140 can reliably support the head brace apparatus 510. Moreover, the movement of the connecting block can be manually driven by medical staff, or can also be driven by the rotation of a rotating power source. It is to be noted that the improvement of the present disclosure lies in that the head brace fixing apparatus 200 fixes the head brace apparatus 510, and the joint locking apparatus 140 is merely the component that connects the head brace fixing apparatus 200 and the head brace apparatus 510, which will not be described in detail here.

Referring to FIGS. 6-10, in an embodiment, the telescoping assembly 210 includes a support arm 211 and a linear motion member 212 slidably connected to the support arm 211 and the support frame 300. The support arm 211 can move with respect to the support frame 300 through the linear motion member 212. The support arm 211 plays the role of carrying and supporting, and provides reliable support for the head brace apparatus 510. An end portion of the support arm 211 is connected to the head brace apparatus 510, and the head brace apparatus 510 is reliably supported by the support arm 211, thereby ensuring that the head brace apparatus 510 is fixed reliably to precisely carry the head 610 of the patient 600, and ensuring the surgical accuracy. The linear motion member 212 is configured to output a linear motion. One end of the linear motion member 212 is fixed to the support frame 300, and the other end of the linear motion member 212 is connected to the support arm 211. The linear motion member 212 can drive the support arm 211 to extend out of the support frame 300 and move to approach the surgical bed 520, and can also drive the support arm 211 to retract into the support frame 300.

Referring to FIGS. 6-10, in an embodiment, the linear motion member 212 includes a third guide member 2121 and a slide member 2122 slidingly engaged with the third guide member 2121. The third guide member 2121 is provided along the axial direction of the support arm 211, and the slide member 2122 is arranged with the support frame 300. When moving along the slide member 2122, the third guide member 2121 can output linear motion and drive the support arm 211 to extend or retract. Through the cooperation of the third guide member 2121 and the slide member 2122, a quick connection between the support arm 211 and the head brace apparatus 510 can be achieved, and the surgical operation time can be reduced. Optionally, the third guide member 2121 is a slide rail, and the slide member 2122 is a slide block. It should be understood that, in other embodiments of the present disclosure, the third guide member 2121 may also be a slide block, and the slide member 2122 may be a slide rail. It should be understood that, in other embodiments of the present disclosure, the linear motion member 212 may include a structure capable of outputting the linear motion, such as a telescopic rod, a rack and pinion structure, or a sprocket structure. It should be noted that the axial direction of the support arm 211 refers to the length direction of the support arm 211.

Optionally, the movement of the third guide member 2121 along the slide member 2122 can be driven manually. Exemplarily, when using the telescoping assembly 210, the medical staff can directly drag the support arm 211, and the support arm 211 moves along the slide member 2122 through the slide member 2122 and extends out of the support frame 300. After use, the medical staff pushes the support arm 211, and the support arm 211 moves along the slide member 2122 through the slide member 2122 and extends out of the support frame 300. It should be understood that the movement of the third guide member 2121 along the slide member 2122 may be driven by a linear power source such as a motor.

In an embodiment, there are two slide blocks, and the two slide blocks are spaced along an extension direction of the slide rail. The slide rail can slide and engage with the two slide blocks simultaneously to ensure an accurate movement trajectory of the slide rail and avoid deflection of the slide rail. Moreover, the two slide blocks can also increase the rigidity of the linear moving member 212 to ensure the carrying capacity of the support arm 211, and reduce the mounting space in the vertical direction to reduce the overall volume.

In an embodiment, there may be at least two linear motion members 212, and the at least two linear motion members 212 are arranged side by side. The at least two linear motion members 212 can be reliably connected to the support arm 211 to improve the carrying capacity of the support arm 211 and further increase the strength of the head brace fixing apparatus 200.

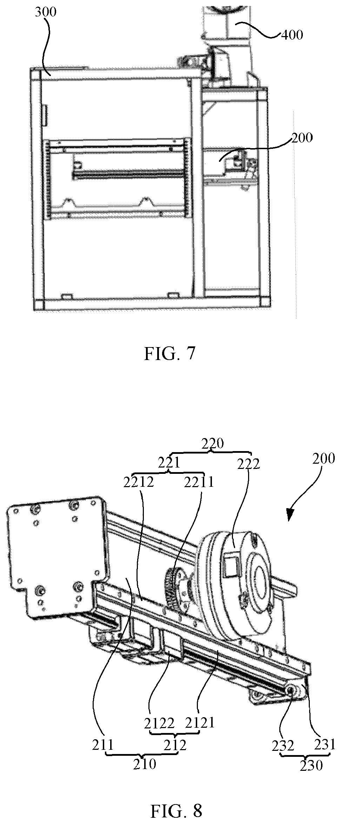

Referring to FIGS. 8-10, in an embodiment, the second locking assembly 220 includes a transmission member 221 and a braking member 222. The braking member 222 is arranged on the support frame 300, and the braking member 222 is connected to the support arm 211 through the transmission member 221. The braking member 222 unlocks or locks the transmission member 221. When the braking member 222 unlocks the transmission member 221, the transmission member 221 can move with the support arm 211. The transmission member 221 has unlocking and locking function. When the transmission member 221 is unlocked, the transmission member 221 can move synchronously with the support arm 211. When the transmission member 221 is locked, the transmission member 221 locks the support arm 211 and limits the telescopic movement of the support arm 211, so that the support arm 211 can be reliably fixed. After the support arm 211 is connected to the head 1421 brace apparatus, the transmission member 221 can fix the support arm 211 in position, and then the head brace apparatus 510 can be reliably fixed.

Moreover, the transmission member 221 is connected to the braking member 222, and the locking and unlocking of the transmission member 221 is controlled by the braking member 222. The braking member 222 is connected to the support frame 300, and an output shaft 2221 of the braking member 222 is movably mounted with the transmission member 221. The transmission member 221 can move within the support arm 211. When the braking member 222 locks the transmission member 221 through the output shaft 2221, the transmission member 221 cannot move, and the transmission member 221further locks the support arm 211 and limit the movement of the support arm 211, so that the support arm 211 can fix the head brace apparatus 510. When the braking member 222 unlocks the transmission member 221, the support arm 211 moves along the slide member 2122 through the third guide member 2121, and the transmission member 221 and the braking member 222 can be driven to move with respect to the braking member 222 through the output shaft 2221.

In an embodiment, the transmission member 221 includes a locking gear 2211 and a locking rack 2212 engaged with the locking gear 2211. The locking gear 2211 is rotatably connected to the braking member 222, and the locking rack 2212 is provided in an axial direction of the support arm 211. When the braking member 222 locks the locking gear 2211, the locking gear 2211 locks the locking rack 2212. The locking gear 2211 is mounted on the output shaft 2221 of the braking member 222, and the locking gear 2211 rotates with respect to the braking member 222 through the output shaft 2221. When the braking member 222 locks the output shaft 2221, the locking gear 2211 cannot rotate. When the braking member 222 unlocks the locking gear 2211, the support arm 211 moves telescopically along the slide member 2122 through the third guide member 2121. At this point, the support arm 211 drives the locking gear 2211 to rotate through the locking rack 2212. When the braking member 222 locks the output shaft 2221, the output shaft 2221 is fixed, so that the locking gear 2211 cannot rotate with respect to the braking member 222. In such a manner, the locking gear 2211 cannot move along the locking rack 2212, and the locking gear 2211 can lock the locking rack 2212 and limit the telescopic movement of the locking rack 2212, so that the support arm 211 is fixed, and then the head brace apparatus 510 is fixed.

In an embodiment, a mounting groove is provided on one side of the support arm 211. The locking rack 2212 is mounted on an inner wall of the mounting groove, and the locking gear 2211 is located within the mounting groove and engaged with the locking rack 2212. Further, the locking rack 2212 is located on a bottom wall or a top wall of the mounting groove.

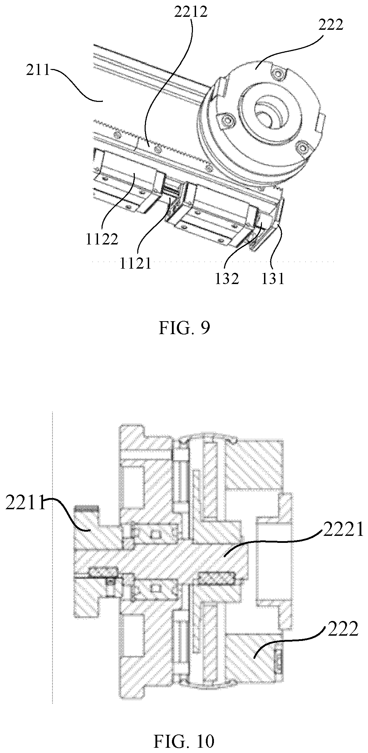

Referring to FIGS. 8 and 10, in an embodiment, the braking member 222 is an electromagnetic brake with a friction plate. When the electromagnetic brake is powered off, the friction plate is in contact with the locking gear 2211. The braking member 222 is electrically connected to a controller of the surgery assisting robot system, which controls power on/off for the braking member 222. When the controller controls the braking member 222 to be powered on, the friction plate is disconnected from the output shaft 2221 of the locking gear 2211, and the locking gear 2211 can rotate freely. When the controller controls the braking member 222 to be powered off, the friction plate abuts against the output shaft 2221 connected to the locking gear 2211 to limit the rotation of the locking gear 2211. Moreover, the controller enables the braking member 222 to be powered on and off, so that both the locking and unlocking of the support arm 211 can be controlled by the medical staff through the controller, thereby reducing the risk of misoperation and improving the safety during a surgical operation.

It should be understood that the braking member 222 can also be a motor, and a motor shaft of the motor is connected to the locking gear 2211. When the motor locks the brake, the motor can lock the locking gear 2211. When the motor releases the brake, the locking gear 2211 can rotate freely. In other embodiments of the present disclosure, the braking member 222 may also be other components capable of fixing the locking gear 2211.

In an embodiment, the head brace fixing apparatus 200 includes a restricting assembly 230 provided on the support arm 211 and configured to restrict the movement of the linear motion member 212. The restricting assembly 230 can restrict a motion stroke of the third guide member 2121 along the slide member 2122 to avoid an overtravel operation of the third guide member 2121, so that the third guide member 2121 is always fitted with the slide member 2122, ensuring the accuracy during the movement process, preventing the third guide member 2121 from being detached from the slide member 2122, and reducing the risk during use of the head brace fixing apparatus 200.

Referring to FIGS. 8 and 9, in an embodiment, the restricting assembly 230 further includes a restricting member 231 provided at the end portion of the support arm 211 and configured to restrict the motion stroke of the third guide member 2121. The restricting members 231 are provided at both ends of the support arm 211 and restrict an extension distance and a retraction distance. When the support arm 211 performs the telescopic movement, the support arm 211 moves along the slide member 2122 by the third guide member 2121. When the restricting member 231 at the end portion of the support arm 211 abuts against the slide member 2122, the support arm 211 cannot continue to move along the slide member 2122 by the third guide member 2121, which indicates that the telescopic movement of the support arm 211 reaches the limit, and the support arm 211 stop continuing to extend or retract.

Moreover, when the second locking assembly 220 cannot reliably lock the support arm 211, the third guide member 2121 can be prevented from detaching from the slide member 2122, thereby reducing the risk during the use of the head brace fixing apparatus 200 and improving the surgery safety. Optionally, the restricting member 231 is a restricting baffle. The restricting baffle is provided at the end portion of the support arm 211 and protrudes from the support arm 211 to facilitate the contact with the slide member 2122.

In an embodiment, the restricting assembly 230 further includes a shock absorber 232 provided on a surface of the restricting member 231 facing the third guide member 2121. The shock absorber 232 can abut against the slide member 2122. The shock absorber 232 plays a buffering role to prevent the slide member 2122 from directly contacting the restricting member 231 in order to avoid damage to parts, which extends the service life of the slide member 2122. When the second locking assembly 220 cannot lock the support arm 211, the slide member 2122 would be likely in contact with the restricting member 231. The shock absorber 232 buffers the slide member 2122 to ensure use safety. Optionally, the shock absorber 232 is a shock-absorbing rubber pad or other components capable of absorbing the shock.

Referring to FIG. 1, the present disclosure also provides a surgery assisting robot system, including a support frame 300, a surgical robotic arm 400 arranged with the support frame 300, and a head brace fixing apparatus 200 as described in the above-mentioned embodiment. The surgical robotic arm 400 is movably arranged with the support frame 300, and the head brace fixing apparatus 200 is arranged with the support frame 300 and can extend and retract with respect to the support frame 300. After extending out of the support frame 300, the head brace fixing apparatus 200 can be connected to the head brace apparatus 510, and fix the head brace apparatus 510. One end of the surgical robotic arm 400 is fixed to the support frame 300, and the other end can clamp a surgical instrument. Optionally, the surgical instrument includes but is not limited to a puncture needle, a scalpel, etc., and may also be other types of handheld instruments.

In the case that the above-mentioned head brace fixing apparatus 200 is used in the surgery assisting robot system of the present disclosure, the head brace apparatus 510 can be reliably connected and fixed to ensure the position of the head 610 of the patient 600 with respect to the surgical robotic arm 400 without occurring any relative displacement, thereby improving the precision of a surgical operation, and ensuring the safety of the surgical operation.

In an embodiment, the surgical robotic arm 400 is a serial robotic arm and/or a parallel robotic arm. In other words, the surgical robotic arm 400 may include a plurality of serial robotic arms, and surgical operations are implemented by connecting the plurality of serial robotic arms. The surgical robotic arm 400 may further include a plurality of parallel robotic arms, and surgical operations are implemented by connecting the plurality of parallel robotic arms. It should be understood that the surgical robotic arm 400 may further include at least one serial robotic arm and at least one parallel robotic arm, and surgical operations are implemented through the cooperation of the serial robotic arm and the parallel robotic arm. In this case, the parallel robotic arm is located at the end of the serial robotic arm. It can be appreciated that the serial robotic arm includes a plurality of single arms, and adjacent single arms are rotatably connected. The parallel robotic arm can include a platform such as a Stewart platform.

### Embodiment III

Referring to FIGS. 1 and 11-14, the present disclosure provides a joint locking apparatus 140. The joint locking apparatus 140 is applied in the surgery assisting robot system and is configured to connect the head brace apparatus 510 of the hospital bed system 500 to the surgery assisting robot system, allowing for connection between the head brace apparatus 510 at any position and the surgery assisting robot system. The head brace apparatus 510 is thus reliably connected and supported, which ensures the accuracy of the connection between the head brace apparatus 510 and the surgery assisting robot system and the precision of a surgical operation. It can be appreciated that the joint locking apparatus 140 can independently provide the connection between the head brace apparatus 510 and the surgery assisting robot system. It can also be appreciated that a plurality of joint locking apparatuses 140 can be provided which are connected to form a head brace connection apparatus 100 that enables the connection to the support frame 300 of the surgery assisting robot system. Moreover, in an embodiment of the present disclosure, the joint locking apparatus 140 is configured to connect the surgery assisting robot system to the head brace apparatus 510. It should be understood that, in other embodiments of the present disclosure, the joint locking apparatus 140 can also provide connection and fixation between other components. The present disclosure merely illustrates the joint locking apparatus 140 configured to connect the surgery assisting robot system to the head brace apparatus 510, as an example.

It can be appreciated that the surgery assisting robot system is used in combination with the hospital bed system 500. The hospital bed system 500 includes a surgical bed 520 and a head brace apparatus 510 provided at one end of the surgical bed 520. The patient 600 lies on the surgical bed 520, and the head 610 of the patient is placed on and supported by the head brace apparatus 510. Further, the head brace apparatus 510 includes a head brace and a support rod supporting the head brace on the surgical bed 520. The support rod plays a supporting role, and the head brace is reliably supported on the surgical bed 520 through the support rod. Moreover, the head brace can be raised and lowered with the surgical bed 520 through the support rod. When the surgical bed 520 collapses, the head brace may also sink together with the surgical bed 520.

The positions of the surgical bed 520 and the surgery assisting robot system may be changed according to specific conditions in an operating room. If the head brace apparatus 510 of the surgical bed 520 cannot be precisely connected to the surgery assisting robot system, the positioning accuracy between the head 610 of the patient 600 and the surgical equipment may be affected, and the position of the head 610 of the patient 600 is not unique, which affects the accuracy of a surgical operation. Therefore, in the present disclosure, the connection between the head brace apparatus 510 and the surgery assisting robot system is provided by the joint locking apparatus 140, and a reliable connection between the head brace apparatus 510 and the surgery assisting robot system is achieved, such that the head 610 of the patient 600 and the surgery assisting robot system are fixed in position. Accordingly, the precision of a surgical operation is ensured, and the accuracy of the surgical operation is improved. The head brace connection apparatus 100 formed by the joint locking apparatus 140 will be described in detail later.

Moreover, the joint locking apparatus 140 has an unlocked position and a locked position. In the locked position, the position of the joint locking apparatus 140 can be adjusted to adapt to the connection between the surgery assisting robot system at different positions and the head brace apparatus 510, in order to facilitate the connection. In the locked position, the joint locking apparatus 140 can fix the connection between the surgery assisting robot system and the head brace apparatus 510, to ensure the reliable fixation between the head brace apparatus 510 and the surgery assisting robot system, in order to fix the head 610 of the patient 600 in position during the later surgical operation.

Referring to FIGS. 1 and 11-14, in an embodiment, the joint locking apparatus 140 includes a guide sleeve 141, a first link 142, a first locking assembly 143 and a second link 144. The guide sleeve 141 is provided with a first mounting hole 1411 and a second mounting hole 1412, axes of which intersect or locate in different planes, that is, the axis of the first mounting hole 1411 and the axis of the second mounting hole 1412 can be arranged in the same (flat) plane and the two corresponding axes may intersect (or be in parallel), or the two corresponding axes may be arranged in different planes. The first link 142 is coaxially connected to the first mounting hole 1411. The first locking assembly 143 is rotatably arranged in the second mounting hole 1412, and the first locking assembly 143 can be locked or unlocked. The second link 144 is connected to the first locking assembly 143 and is rotatably provided in the second mounting hole 1412. The first locking assembly 143 is configured to lock the positions of the first link 142 and the second link 144 with respect to the guide sleeve 141.

The guide sleeve 141 is a fixing body of the joint locking apparatus 140, and the first link 142 and the second link 144 are fixed in their relative positions through the guide sleeve 141. The first mounting hole 1411 and the second mounting hole 1412 are arranged to pass through the guide sleeve 141, and the axis of the first mounting hole 1411 and the axis of the second mounting hole 1412 intersect or are arranged in different planes. In other words, the axis of the first mounting hole 1411 and the axis of the second mounting hole 1412 are arranged apart from each other to avoid interference of components mounted in the first mounting hole 1411 and the second mounting hole 1412. Optionally, there exists an included angle between the first mounting hole 1411 and the second mounting hole 1412 in the three-dimensional space. Further, the first mounting hole 1411 and the second mounting hole 1412 are arranged orthogonally (in different planes) in the three-dimensional space.

The first link 142 is coaxially connected into the first mounting hole 1411, that is, the first link 142 is provided in the first mounting hole 1411, and the first link 142 can move in the axial direction of the first mounting hole 1411 and rotate in the first mounting hole 1411. The second link 144 is rotatably mounted in the second mounting hole 1412. The first locking assembly 143 is also provided in the second mounting hole 1412. An end portion of the first locking assembly 143 protruding from the second mounting hole 1412 is connected to the second link 144. The first locking assembly 143 can drive the second link 144 to rotate. When the joint locking apparatus 140 is in an unlocked position, the first link 142 has a freedom of movement in the axis direction of the first mounting hole 1411 and a freedom of rotation about the axis direction of the first mounting hole 1411, and the second link 144 has a freedom of rotation in the axis direction of the second mounting hole 1412. In such a manner, a connection length and an orientation of the joint locking apparatus 140 can be adjusted to adapt to the distance and orientation between the surgery assisting robot system and the head brace apparatus 510.

Optionally, the first link 142 and the second link 144 have a rod-shaped structure. Optionally, at least one end of the first link 142 is provided with a connector 1421 by which the first link 142 can be connected to a first link 142, a second link 144, or a first locking assembly 143 of an adjacent joint locking apparatus 140. Optionally, at least one end of the second link 144 is provided with a connecting disc 1441 that can be connected to the first link 142, the second link 144, or the first locking assembly 143 of an adjacent joint locking apparatus 140.

The first locking assembly 143 has a locked position and an unlocked position. In the locked position, the first locking assembly 143 locks the first link 142 and the second link 144 to the guide sleeve 141, and the first link 142 and the second link 144 are fixed in position with respect to the guide sleeve 141, such that the first link 142 and the second link 144 cannot move with respect to the guide sleeve 141, and the joint locking apparatus 140 is therefore locked. In the unlocked position, the first locking assembly 143 unlocks the first link 142 and the second link 144 from the guide sleeve 141, such that the first link 142 and the second link 144 can move with respect to the guide sleeve 141, and the joint locking apparatus 140 is therefore unlocked.

In the joint locking apparatus 140 in the above-mentioned embodiment, the guide sleeve 141 and the first locking assembly 143 allow for the locking and unlocking of the first link 142 and the second link 144, which effectively solves the problem that the head brace of the surgical bed 520 cannot be connected to the surgery assisting robot system, and achieves the reliable connection between the head brace apparatus 510 and the surgery assisting robot system. The head 610 of the patient 600 and the surgery assisting robot system can be thus fixed in position, thereby ensuring surgical precision, and improving surgical accuracy.

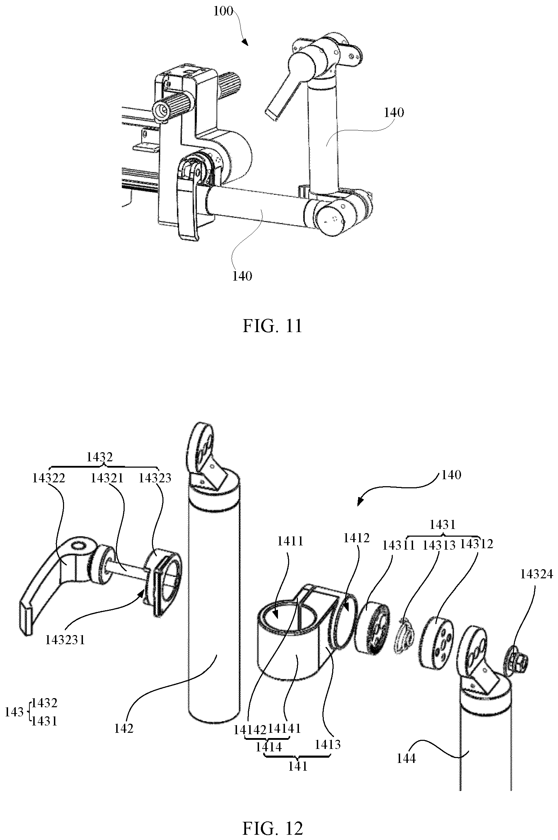

Referring to FIGS. 11-14, in an embodiment, the first locking assembly 143 includes a locking member 1431 and a lever assembly 1432 connected to the locking member 1431. The locking member 1431 is movably provided in the second mounting hole 1412, and the lever assembly 1432 is configured to lock or unlock the locking member 1431. The locking member 1431 is rotatably mounted in the second mounting hole 1412. An end portion of the locking member 1431 protruding from the second mounting hole 1412 is connected to the second link 144, and the first locking assembly 143 can drive the second link 144 to rotate. The lever assembly 1432 is movably provided on the guide sleeve 141 and is connected to the locking member 1431. When moving, the lever assembly 1432 can drive the locking member 1431 to move, and to move with respect to the guide sleeve 141.

The lever assembly 1432 has a locked position and an unlocked position so that the first locking assembly 143 is in the locked position or the unlocked position. When the lever assembly 1432 moves to the locked position, the lever assembly 1432 drives the locking member 1431 to move, so that the locking member 1431 locks the second link 144 to limit a rotation displacement of the second link 144 about the axis direction of the second mounting hole 1412. Meanwhile, the lever assembly 1432 can also abut against the guide sleeve 141, so that the guide sleeve 141 locks the first link 142 to limit the movement displacement of the first link 142 in the axis direction of the first mounting hole 1411 and rotation displacement of the first link 142 about the axis direction of the first mounting hole 1411. At this point, the joint locking apparatus 140 is in the locked position, and the joint locking apparatus 140 does not have any degree of freedom and cannot produce any displacement, thereby ensuring a reliable connection between the surgery assisting robot system and the head brace apparatus 510, ensuring the unique position relationship between the head 610 of the patient 600 and the surgery assisting robot system, and ensuring the positioning accuracy of the head 610 of the patient 600.

When the lever assembly 1432 moves to the unlocked position, the lever assembly 1432 drives the locking member 1431 to move, such that the locking member 1431 unlocks the second link 144, and the second link 144 can rotate in the axis direction of the second mounting hole 1412. Meanwhile, the lever assembly 1432 is detached from the guide sleeve 141, such that the guide sleeve 141 unlocks the first link 142, and the first link 142 can move in the axis direction of the first mounting hole 1411 and rotate about the axis direction of the first mounting hole 1411. At this point, the joint locking apparatus 140 is in the unlocked position, and the joint locking apparatus 140 has multi-directional degrees of freedom and is able to adjust the positions of the first link 142 and the second link 144 with respect to the guide sleeve 141 to adapt to the distance between the surgery assisting robot system and the head brace apparatus 510, thereby ensuring the accurate connection relationship between the head brace apparatus 510 and the surgery assisting robot system.

In an embodiment, the guide sleeve 141 includes a guide body 1413 and a guide pressing plate 1414 connected to the guide body 1413. The guide body 1413 is provided with a second mounting hole 1412. The guide pressing plate 1414 surrounds and forms the first mounting hole 1411, and a preset space exists between the guide pressing plate 1414 and the guide body 1413. When the first locking assembly 143 is in the locked position, the first locking assembly 143 can push the guide pressing plate 1414 to fix the first link 142.

The guide body 1413 is the main portion of the guide sleeve 141 for connecting and fixing. The guide body 1413 has a through second mounting hole 1412, the locking member 1431 of the first locking assembly 143 is located in the second mounting hole 1412, and the lever assembly 1432 of the first locking assembly 143 partially extends into the second mounting hole 1412 and connects to the locking member 1431, driving the locking member 1431 to move and lock or unlock the second link 144. One end of the guide pressing plate 1414 is connected to the guide body 1413, and the other end of the guide pressing plate 1414 is a free end. An inner wall of the guide pressing plate 1414 and the guide body 1413 surround and form the first mounting hole 1411.

There exists a preset distance between the guide pressing plate 1414 and the guide body 1413. When the guide pressing plate 1414 moves away from the guide body 1413, i.e., when the preset distance is increased, an aperture size of the first mounting hole 1411 is increased. When the guide pressing plate 1414 moves toward the guide body 1413, i.e., when the preset distance is reduced, the aperture size of the first mounting hole 1411 is reduced. In other words, the preset distance can enable the adjustment of the tightness between the first mounting hole 1411 and the first link 142. In such a manner, when the guide pressing plate 1414 moves toward the guide body 1413, i.e., when the preset distance is reduced, the guide pressing plate 1414 may press the first link 142, so that the first link 142 cannot move in the first mounting hole 1411, and the first link 142 is locked. When the preset distance returns to normal or becomes larger, the first link 142 can move in the first mounting hole 1411.

When the first locking assembly 143 is in the locked position, the first locking assembly 143 abuts against the guide pressing plate 1414 to push the guide pressing plate 1414 to move toward the guide body 1413, so that the guide pressing plate 1414 presses the first link 142. At this point, the pressing force applied by the guide pressing plate 1414 to the first link 142 and the friction force between the guide pressing plate 1414 and the first link 142 limits the movement of the first link 142 in the first mounting hole 1411, so that the first link 142 is reliably fixed in the first mounting hole 1411. Optionally, a diameter of the first mounting hole 1411 is greater than or equal to the diameter of the first link 142, which facilitates the mounting of the first link 142 in the first mounting hole 1411 and facilitates the movement and rotation of the first link 142 in the first mounting hole 1411. It should be understood that, in other embodiments of the present disclosure, the diameter of the first mounting hole 1411 may be less than the diameter of the first link 142.

Optionally, the guide body 1413 and the guide pressing plate 1414 are formed in one piece which can reduce the number of parts and reduce the assembly steps, providing ease of use. Meanwhile, the reliability of the connection between the guide body 1413 and the guide pressing plate 1414 can be ensured, and the reliable operation of the guide body 1413 and the guide pressing plate 1414 can be ensured.

Optionally, the inner wall of the second mounting hole 1412 has a limit protrusion, the limit protrusion protrudes in a radial direction of the second mounting hole 1412, and the limit protrusion is located at an end of the second mounting hole 1412 facing the lever assembly 1432. After the locking member 1431 is mounted in the second mounting hole 1412, one end of the locking member 1431 abuts against the limit protrusion, and the other end of the locking member 1431 is connected to the second link 144. The displacement of the locking member 1431 in the axial direction of the second mounting hole 1412 is limited by the limit protrusion. Moreover, when the lever assembly 1432 drives the locking member 1431 to lock, the locking member 1431 can be reliably locked by a stop function of the limit protrusion. Meanwhile, the lever assembly 1432 can also push the guide pressing plate 1414 to move toward the guide body 1413, to lock the first link 142.

Referring to FIGS. 11-14, in an embodiment, the guide pressing plate 1414 includes a guide curved plate 14141 connected to the guide body 1413, and a guide fixing plate 14142 connected to the guide curved plate 14141. The guide curved plate 14141 surrounds and forms the first mounting hole 1411. The guide fixing plate 14142 is parallel to the surface of the guide body 1413, and there is a preset distance between the guide fixing plate 14142 and the guide body 1413. One end of the guide curved plate 14141 is connected to the guide body 1413, and the other end of the guide curved plate 14141 is connected to the guide fixing plate 14142. The guide curved plate 14141 and the guide body 1413 surround and form the first mounting hole 1411. The guide fixing plate 14142 is a flat plate and is parallel to an end surface of the second mounting hole 1412. The lever assembly 1432 extends through the guide fixing plate 14142 and is connected to the first locking assembly 143 in the second mounting hole 1412.

When the lever assembly 1432 is in the locked position, the lever assembly 1432 abuts against the guide fixing plate 14142, and pushes the guide fixing plate 14142 to move toward the guide body 1413, so that the guide curved plate 14141 presses the first link 142. At this point, the guide curved plate 14141 is tightly fitted with the first link 142, and the first link 142 cannot move in the first mounting hole 1411. The first link 142 is therefore fixed. When the lever assembly 1432 is in the unlocked position, the lever assembly 1432 is detached from the guide fixing plate 14142, and the guide fixing plate 14142 returns. At this point, the guide curved plate 14141 is loosely fitted with the first link 142, and the first link 142 can move in the first mounting hole 1411. The first link 142 is therefore unlocked.

Optionally, the guide curved plate 14141 and the guide fixing plate 14142 are formed in one piece, which can reduce the number of parts and reduce the assembly steps, providing ease of use. Meanwhile, the reliability of the connection between the guide curved plate 14141 and the guide fixing plate 14142 can be ensured, and the reliable operations of the guide curved plate 14141 and the guide fixing plate 14142 can be ensured.

Optionally, the shape of the guide body 1413 is essentially unlimited. The guide body 1413 can be a guide block, a guide seat, etc., as long as the guide body 1413 can be provided with the second mounting hole 1412, and have a certain carrying strength. Exemplarily, the guide body 1413 has a spliced structure formed by a square structure and a cylindrical structure. It should be understood that, in other embodiments of the present disclosure, the guide body 1413 can also have a square shape, a prism shape, etc.

Referring to FIGS. 11-14, in an embodiment, the locking member 1431 includes a first locking disc 14311, a second locking disc 14312, and a second elastic member 14313 provided between the first locking disc 14311 and the second locking disc 14312. The lever assembly 1432 is configured to lock or unlock the first locking disc 14311 and the second locking disc 14312. The elastic force of the second elastic member 14313 allows the first locking disc 14311 and the second locking disc 14312 to be away from each other. When the lever assembly 1432 is in the locked position, the lever assembly 1432 allows the first locking disc 14311 and the second locking disc 14312 to compress the second elastic member 14313such that the first locking disc 14311 and the second locking disc 14312 are engaged with and locked to each other.

After the first locking disc 14311 and the second locking disc 14312 are mounted in the second mounting hole 1412, the first locking disc 14311 is fixed in the second mounting hole 1412, and the second locking disc 14312 is connected to the second link 144. After the first locking disc 14311 is engaged with the second locking disc 14312, the first locking disc 14311 restricts the rotational movement of the second locking disc 14312, and further restricts the second link 144 so that the second link 144 cannot rotate. When the second locking disc 14312 disengages from the first locking disk 14311, the second locking disk 14312 and the second link 144 can rotate within the second mounting hole 1412.

The second elastic member 14313 is located between the first locking disc 14311 and the second locking disc 14312. The elastic force of the second elastic member 14313 allows the first locking disc 14311 and the second locking disc 14312 to be away from each other, such that the second link 144 remains in the unlocked state. When the lever assembly 1432 is in the locked position, the lever assembly 1432 can drive the second locking disc 14312 to compress the second elastic member 14313, such that the second locking disc 14312 approaches the first locking disc 14311 and is engaged with the first locking disc 14311. At this point, the first locking disc 14311 locks the second link 144 with the help of the second locking disc 14312. When the lever assembly 1432 moves to the unlocked position, the external force on the second locking disc 14312 is released, and the elastic force of the second elastic member 14313 pushes the second locking disc 14312 to disengage from the first locking disc 14311. Optionally, the second elastic member 14313 is a spring. Optionally, there is at least one second elastic member 14313.

In an embodiment, the first locking disc 14311 has a first locking portion, and the second locking disc 14312 has a second locking portion. When the first locking disc 14311 is engaged with and locked to the second locking disc 14312, the first locking portion and the second locking portion engage and lock each other. The first locking portion is provided on the surface of the first locking disc 14311 facing the second locking disc 14312, and the second locking portion is provided on the surface of the second locking disc 14312 facing the first locking disc 14311. When the first locking disc 14311 is engaged with the second locking disc 14312, the first locking portion engages the second locking portion, and the first locking portion can limit the rotation of the second locking portion, thereby limiting the rotation of the second link 144 driven by the second locking disc14312. The second link 144 is thereby locked.

Optionally, the first locking portion and the second locking portion are tooth-shaped. Exemplarily, the first locking portion and the second locking portion are tooth portions that engage with each other, and the rotation of the second locking disc 14312 is limited by the tooth portions that engage with each other. Optionally, the first limit portion engages the second limit portion in a manner that a protrusion engages a recess. Exemplarily, the first locking portion is a protrusion, and the second locking portion is a groove. The rotation of the second locking disc 14312 can be limited when the protrusion engages the groove. It should be understood that the first locking portion and the second locking portion can also be components that are able to limit the rotation of the second locking disc 14312, such as a restricting post and a hook that are arranged in a staggered manner.

In an embodiment, the lever assembly 1432 includes a connecting shaft 14321 and a locking lever 14322 rotatably connected to the connecting shaft 14321. The connecting shaft 14321 extends through the guide pressing plate 1414 and the guide body 1413 and is connected to the first locking disc 14311 and the second locking disc 14312. When the locking lever 14322 is in the locked position, the locking lever 14322 drives the guide pressing plate 1414 to abut against the guide body 1413 through the connecting shaft 14321, and allows the first locking disc 14311 and the second locking disc 14312 to engage and lock each other. The connecting shaft 14321 is movably connected to the guide pressing plate 1414, the guide body 1413, the first locking disc 14311, and the second locking disc 14312.

The locking lever 14322 can be operated with respect to the connecting shaft 14321. The locking lever 14322 can move to the locked position and the unlocked position with respect to the locking lever 14322. When the locking lever 14322 is operated to the locked position, the locking lever 14322 drives the connecting shaft 14321 to move away from the second link 144 in the axis direction of the second mounting hole 1412. Meanwhile, the connecting shaft 14321 drives the second locking disc 14312 to compress the second elastic member 14313 and to move toward the first locking disc 14311, to lock the second locking disc 14312. Meanwhile, the locking lever 14322 pushes the guide pressing plate 1414 to move toward the guide body 1413, to lock the first link 142.

When the locking lever 14322 is operated to the unlocked position, the locking lever 14322 drives the connecting shaft 14321 to move towards the second link 144 in the axis direction of the second mounting hole 1412. At this point, the second locking disc 14312 is detached from the first locking disc 14311 under the elastic force of the second elastic member 14313, and the second link 144 is unlocked. Meanwhile, the locking lever 14322 is detached from the guide pressing plate 1414, and the guide pressing plate 1414 returns to unlock the first link 142.

Optionally, the connecting shaft 14321 can also be connected to the second link 144. It should be understood that the second locking disc 14312 can also be connected to the second link 144 independently. Optionally, the lever assembly 1432 further includes a nut member 14324 provided at the end of the connecting shaft 14321, and configured to fix various components on the connecting shaft 14321. Moreover, the nut member 14324 can also adjust the distance between the first locking disc 14311 and the second locking disc 14312, and adjust the preset distance between the guide body 1413 and the guide pressing plate 1414, to adjust the operating force for operating the locking lever 14322. Further, the nut member 14324 is a double nut, which can prevent the nut member 14324 from loosening and ensure the stability of the entire structure.

In an embodiment, an end portion of the locking lever 14322 connected to the connecting shaft 14321 has a cam shape. In other words, the locking lever 14322 abuts against the guide pressing plate 1414 through a cam portion to push the guide pressing plate 1414 to move towards the guide body 1413. Specifically, when the cam portion of the locking lever 14322 abuts against the guide pressing plate 1414, due to the limiting effect of the first locking disc 14311, the locking lever 14322 is fixed in position, and the cam portion can only push the guide pressing plate 1414 to move towards the guide body 1413. When the cam portion of the locking lever 14322 is detached from the guide pressing plate 1414, the guide pressing plate 1414 since there is no external force applied thereto.

Optionally, when the locking lever 14322 is in the locked position, the locking lever 14322 is orthogonal to the first link 142 and the second link 144 in space, which can reduce the space required for unlocking of the locking lever 14322, and avoid the situation where the locking lever 14322 cannot be unlocked due to a too small space among the locking lever 14322, the first link 142 and the second link 144, providing ease of use.

In another embodiment, the locking member 1431 includes a second locking disc 14312, a second elastic member 14313 provided between the second locking disc 14312 and the second mounting hole 1412 A circular protrusion is provided in the second mounting hole 1412, and the second locking disk 14312 is locked and engaged with the circular protrusion. A tooth-shaped structure is provided on the surface of the circular protrusion that engages with the second locking disc 14312. The second locking portion engages with the tooth-shaped structure. When the second locking portion abuts against the tooth-shaped structure, the rotation of the second locking disc 14312 is locked, and the rotation of the second link 144 is thereby locked. The elastic force of the second elastic member 14313 allows the second locking disc 14312 to move away from the circular protrusion of the second mounting hole 1412.

In another embodiment of the present disclosure, the locking member 1431 includes a second elastic member 14313 provided between the second link 144 and the second mounting hole 1412. A circular protrusion is provided in the second mounting hole 1412. A mounting protrusion is provided at the end of the second link 144 which is mounted to the second mounting hole 1412, and matches the second mounting hole 1412. The circular protrusion is locked and engaged with the mounting protrusion. The tooth-shaped structures are respectively provided on the surfaces of the circular protrusion and the locking protrusion which are in contact with each other. When the circular protrusion abuts against the locking protrusion, the tooth-shaped structure of the circular protrusion engages with the tooth-shaped structure of the locking protrusion, to lock the rotation of the second link 144. The elastic force of the second elastic member 14313 allows the circular protrusion of the second mounting hole 1412 to move away from the mounting protrusion of the second link 144.

In an embodiment, the lever assembly 1432 further includes a stopper 14323 which sleeves over the connecting shaft 14321 and is provided on the guide sleeve 141. The stopper 14323 is configured to limit the position of the locking lever 14322 when the locking lever is locked. The stopper 14323 is connected to the guide pressing plate 1414. When the locking lever 14322 is in the locked position, the locking lever 14322 is located within the stopper 14323, and the rotation of the locking lever 14322 is limited by the stopper 14323 to ensure accurate locking.

It should be appreciated that when the locking lever 14322 rotates around the connecting shaft 14321, the locking lever 14322 can move into or out of the stopper 14323. When the locking lever 14322 moves from the unlocked position to the locked position, the locking lever 14322 moves into the stopper 14323. The rotation of the locking lever 14322 is limited by the stopper 14323, so that the locking lever 14322 is accurately located at the locked position, and the locking lever 14322 keeps perpendicular to the first link 142 and the second link 144, which is convenient for the later unlocking operation. When the locking lever 14322 moves from the locked position to the unlocked position, the locking lever 14322 moves out of the stopper 14323.

Optionally, the stopper 14323 is a structure of a mounting seat or a mounting platform, and is configured to mount the locking lever 14322. In an embodiment, the stopper 14323 has a restricting gap 143231. When the locking lever 14322 is locked, the locking lever 14322 is located within the restricting gap 143231. By the inner wall of the restricting gap 143231 stopping the locking lever 14322, the locking lever 14322 is reliably located within the restricting gap 143231 when the locking lever 14322 is in the locked position. It should be understood that, in other embodiments of the present disclosure, restricting components such as a restricting post may also be provided on the stopper 14323.

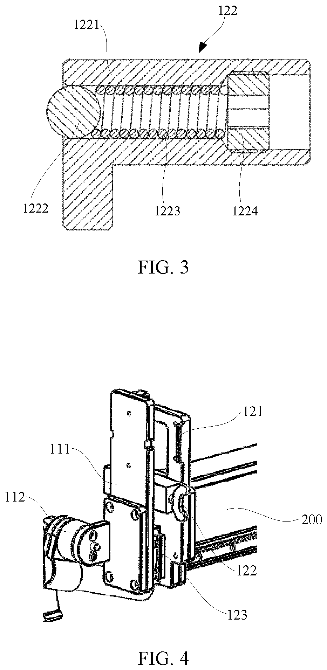

The mechanism of the joint locking apparatus 140 in the present disclosure is described below. Referring to FIGS. 3 and 4, when the locking lever 14322 is operated to the locked position, the locking lever 14322 is pressed tightly, and drives the second locking disc 14312 to compress the second elastic member 14313, move towards the first locking disc 14311, and then engage with and lock to the first locking disc 14311. The relative rotations of the first locking disc 14311 and the second locking disc 14312 are thereby limited, and further, the second link 144 is locked. Meanwhile, the locking lever 14322 presses the guide pressing plate 1414 through the cam portion, so that the guide pressing plate 1414 moves toward the guide body 1413. At this point, the guide pressing plate 1414 presses the first link 142 to lock the first link 142.

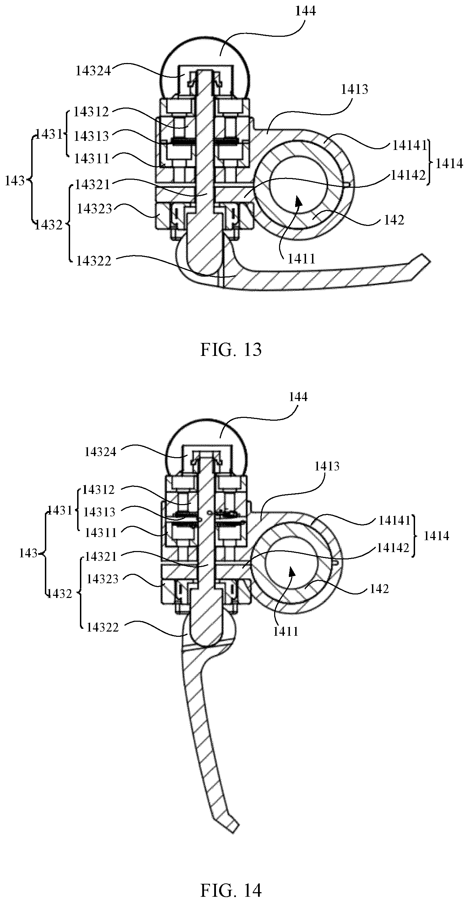

Referring to FIGS. 12 and 14, when the locking lever 14322 is operated to the unlocked position, the locking lever 14322 is raised up, and the external force on the second locking disc 14312 is released. The second locking disc 14312 moves away from the first locking disc 14311 under the elastic force of the second elastic member 14313. The first locking disc 14311 and the second locking disc 14312 can rotate with respect to each other. The second link 144 is unlocked and rotates within the second mounting hole 1412. Meanwhile, the locking lever 14322 is detached from the guide pressing plate 1414, the guide pressing plate 1414 returns, and a normal preset distance is restored. The first link 142 is thus unlocked and rotates and moves in the first mounting hole 1411.

Moreover, the locking lever 14322 is in the unlocked position, the first link 142 can rotate and move, and the second link 144 can rotate, such that the joint locking apparatus 140 has three degrees of freedom in three directions. The above three degrees of freedom can be locked when the locking lever 14322 is in the locked position. The joint locking apparatus 140 of the present disclosure can provide fast locking of the first link 142 and the second link 144, facilitating quick fixation of the head 610 of the patient 600 by the medical staff, and providing ease of use.

Referring to FIGS. 1 and 11-13, the present disclosure further provides a head brace connection apparatus 100 including a plurality of joint locking apparatuses 140 as described in the above-mentioned embodiments. The plurality of joint locking apparatuses 140 are connected in series. The first link 142 of one of the joint locking apparatuses 140 is connected to the first link 142 or the second link 144 of an adjacent joint locking apparatus 140. The second link 144 of one of the joint locking apparatuses 140 is connected to the second link 144 or the first link 142 of an adjacent joint locking apparatus 140. In other words, the connection relationship between the head brace apparatus 510 and the support frame 300 of the surgery assisting robot system is established by forming the head brace connection apparatus 100 with a plurality of joint locking apparatuses 140. It can be appreciated that in the case that the distance between the head brace apparatus 510 on the surgical bed 520 and the support frame 300 of the surgery assisting robot system is large, a single joint locking apparatus 140 may not be able to provide an accurate connection therebetween, and in this case, by forming the head brace connection apparatus 100 with the plurality of joint locking apparatuses 140, the connection length can be ensured, and also, multiple degrees of freedom is provided to meet the requirements for connections in different directions.

The first joint locking apparatus 140 of the head brace connection apparatus 100 is connected to the support frame 300, and the last joint locking apparatus 140 of the head brace connection apparatus 100 is connected to the head brace apparatus 510. Two adjacent joint locking apparatuses 140 are connected in series. As such, the support frame 300 of the surgery assisting robot system can be connected to the head brace apparatus 510 of the hospital bed system 500. Exemplarily, the head brace connection apparatus 100 includes two joint locking apparatuses 140. When connected in series, the two joint locking apparatuses 140 generate six degrees of freedom in six directions, which can satisfy any positioning requirements of the surgery assisting robot system and the head brace apparatus 510. It should be understood that, in other embodiments of the present disclosure, the number of joint locking apparatuses 140 may be three, four, or even more.

The head brace connection apparatus 100 of the present disclosure can precisely connect the support frame 300 of the surgery assisting robot system and the head brace apparatus 510 of the hospital bed system 500, such that the relative position of the head 610 of the patient 600 with respect to the surgery assisting robot system does not change, which is convenient for positioning the head 610 of the patient 600, ensuring accuracy throughout a surgical operation.

The present disclosure further provides a surgery assisting robot system, including a support frame 300, a surgical robotic arm 400 arranged with the support frame 300, and the head brace connection apparatus 100 in the above-mentioned embodiments. The surgical robotic arm 400 is movably arranged with the support frame 300, and the head brace connection apparatus 100 is arranged with the support frame 300 and can move with respect to the support frame 300, to allow the head brace connection apparatus 100 to connect and fix the head brace apparatus 510. One end of the surgical robotic arm 400 is fixed to the support frame 300, and the other end can clamp the surgical instrument. Optionally, the surgical instrument includes but is not limited to a puncture needle, a scalpel, etc., and may also be other types of handheld instruments.

After the surgery assisting robot system of the present disclosure adopts the above-mentioned head brace connection apparatus 100, the head brace apparatus 510 can be reliably connected and fixed, to ensure the relative positions of the head 610 of the patient 600 and the surgical robotic arm 400 without occurring any relative displacements, thereby improving the surgical precision, and ensuring the safety of a surgical operation.

In an embodiment, the surgical robotic arm 400 is a serial robotic arm and/or a parallel robotic arm. In other words, the surgical robotic arm 400 may include a plurality of serial robotic arms, and surgical operations are implemented by connecting the plurality of serial robotic arms. The surgical robotic arm 400 may further include a plurality of parallel robotic arms, and surgical operations are implemented by connecting the plurality of parallel robotic arms. It should be understood that the surgical robotic arm 400 may further include at least one serial robotic arm and at least one parallel robotic arm, and surgical operations are implemented through the cooperation of the serial robotic arm and the parallel robotic arm. In this case, the parallel robotic arm is located at the end of the serial robotic arm. It can be appreciated that the serial robotic arm includes a plurality of single arms, and adjacent single arms are rotatably connected. The parallel robotic arm can include a platform such as a Stewart platform.

### Embodiment IV

It is to be noted that in the surgery assisting robot system of the above-mentioned three embodiments, the support unlocking apparatus 100, the joint locking apparatus 140 and the head brace fixing apparatus 200 can be used separately (see the above-mentioned embodiments for details), or can be used in combination. The combination here may include a combination of two of the above, or a combination of three of the above. The specific structures of the support unlocking apparatus 100, the joint locking apparatus 140 and the head brace fixing apparatus 200 will not be repeated in this embodiment. In the embodiment IV, the head brace apparatus 510 supported by the combination of the support unlocking apparatus 100, the joint locking apparatus 140 and the head brace fixing apparatus 200 is taken as an example to illustrate the support for the head brace apparatus 510.

The head brace fixing apparatus 200 is movably provided in the support frame 300 of the surgery assisting robot system. The support unlocking apparatus 100 is mounted on the head brace fixing apparatus 200 and includes the joint locking apparatus 140. When needing to support the head brace apparatus 510, the head brace fixing apparatus 200 extends out of the support frame 300, so that the support unlocking apparatus 100 is connected to the head brace apparatus 510. In the process of connecting the support unlocking apparatus 100 to the head brace apparatus 510, the joint locking apparatus 140 is utilized to lock and unlock multiple links to adjust the position of the support unlocking apparatus, so that the joint locking apparatus 140 in the support unlocking apparatus 100 can be connected to the head brace apparatus 510. After the surgery is completed,

If the surgical bed 520 collapses during a surgical operation, the surgical bed 520 may generate a sinking force that drags the head brace apparatus 510, such that the head brace apparatus 510 is subjected to a drag force. The drag force is then transferred to the clamping apparatus 120 through the adapter assembly 112 and the mounting plate 111. Since the sinking of the surgical bed 520 produces a larger drag force on the head brace apparatus 510, which exceeds the threshold of the clamping force of the clamping apparatus 120, the clamping apparatus 120 cannot clamp the mounting plate 111. So, the mounting plate 111 sinks synchronously with the surgical bed 520 with respect to the clamping apparatus 120 under the drag force, and the head 610 of the patient 600 sinks together with the surgical bed 520 to avoid dragging the head 610 of the patient 600.

The technical features of the above-mentioned embodiments can be combined arbitrarily. In order to make the description concise, all possible combinations of the technical features in the above-mentioned embodiments are not described. However, as long as there is no contradiction in the combination of these technical features, they should be considered as the scope of the present disclosure.

The above-mentioned embodiments are merely some embodiments of the present disclosure, and the descriptions thereof are relatively specific and detailed, but should not be construed as limiting the scope of the present disclosure. It should be noted that those skilled in the art can make several modifications and improvements without departing from the concept of the present disclosure, and these all belong to the protection scope of the present disclosure. Therefore, the scope of protection of the patent disclosure should be subject to the appended claims.

## Claims

1. A support unlocking apparatus, provided on a head brace fixing apparatus of a surgery assisting robot system and connected to a head brace apparatus of a hospital bed system, comprising:
a joint locking apparatus, comprising an adapter assembly connected to the head brace apparatus and a mounting plate configured to mount the adapter assembly; and
a clamping apparatus, provided on the head brace fixing apparatus and configured to clamp the mounting plate, the clamping apparatus having an unlocked state and a locked state, the clamping apparatus being placed in the unlocked state and the mounting plate moving with respect to the clamping apparatus when a drag force applied to the joint locking apparatus exceeds a threshold of the clamping apparatus, and the mounting plate being fixed in the clamping apparatus when the clamping apparatus is in the locked state.

2. The support unlocking apparatus according to claim 1, wherein the clamping apparatus comprises a connecting plate and a clamping assembly, the connecting plate being mounted on the head brace fixing apparatus, the clamping assembly being provided on the connecting plate and clamping the mounting plate.

3. The support unlocking apparatus according to claim 2, wherein the clamping assembly comprises a fixing bracket, a first elastic member provided in the fixing bracket, and an abutting member provided at an end portion of the first elastic member, the fixing bracket being connected to the connecting plate, and the abutting member abutting against the mounting plate under an elastic force of the first elastic member.

4. The support unlocking apparatus according to claim 3, wherein the clamping assembly further comprises an adjustment member movably provided in the fixing bracket and abutting against the first elastic member, the adjustment member being configured to adjust the elastic force of the first elastic member.

5. The support unlocking apparatus according to claim 3, wherein the mounting plate includes a restricting recess engageable with the abutting member, and the abutting member is capable of moving into or out of the restricting recess.

6. The support unlocking apparatus according to any one of claims 3 to 5, wherein the clamping assembly comprises a plurality of clamping assemblies arranged symmetrically on both sides of the mounting plate, and each side of the mounting plate is provided with at least one clamping assembly.

7. The support unlocking apparatus according to claim 2, wherein the clamping assembly comprises a clamping sleeve provided on the connecting plate, a locking member movably provided in the clamping sleeve, and a pressing tab provided at an end portion of the locking member, the pressing tab abutting against the mounting plate, and the locking member being configured to adjust an abutting force between the pressing tab and the mounting plate.

8. The support unlocking apparatus according to claim 2, wherein the clamping apparatus further comprises a guide assembly, the mounting plate being connected to the connecting plate through the guide assembly, and the mounting plate being movable with respect to the connecting plate through the guide assembly, and
wherein the guide assembly comprises a first guide member and a second guide member, the first guide member being provided on the mounting plate, the second guide member being provided on the connecting plate, and the first guide member slidingly engaging with the second guide member.

9. The support unlocking apparatus according to any one of claims 2, 3 or 7, wherein the clamping apparatus further comprises a friction member provided on the connecting plate and abutting against the mounting plate.

10. A surgery assisting robot system, comprising a support frame, a surgical robotic arm arranged with the support frame, a head brace fixing apparatus, and a support unlocking apparatus according to any one of claims 1 to 9,
wherein the support unlocking apparatus is provided on the head brace fixing apparatus, and the head brace fixing apparatus is configured to support a head brace apparatus through the support unlocking apparatus, the surgical robotic arm being configured to perform a surgical operation, and the support frame being configured to control movements of the surgical robotic arm and the head brace fixing apparatus.

11. Ajoint locking apparatus, comprising:
a guide sleeve having a first mounting hole and a second mounting hole, axes of which intersect or are located in different planes;
a first link coaxially connected to the first mounting hole;
a second link rotatably provided within the second mounting hole; and
a first locking assembly, provided in the second mounting hole and configured to lock the first link and the second link in position with respect to the guide sleeve.

12. The joint locking apparatus according to claim 11, wherein the guide sleeve comprises a guide body and a guide pressing plate connected to the guide body, the second mounting hole being provided in the guide body, the guide pressing plate surrounds and forms the first mounting hole, and a preset distance existing between the guide pressing plate and the guide body.

13. The joint locking apparatus according to claim 12, wherein the guide pressing plate comprises a guide curved plate connected to the guide body and a guide fixing plate connected to the guide curved plate, and the guide curved plate surrounds and forms the first mounting hole, the guide fixing plate being parallel to a surface of the guide body with the preset distance between the guide fixing plate and the guide body.

14. The joint locking apparatus according to claim 12 or 13, wherein the first locking assembly comprises a locking member and a lever assembly connected to the locking member, the locking member being movably provided in the second mounting hole, and the lever assembly being configured to lock or unlock the locking member, and
wherein the locking member comprises a first locking disc, a second locking disc, and an elastic member provided between the first locking disc and the second locking disc, the lever assembly being configured to lock or unlock the first locking disc and the second locking disc.

15. The joint locking apparatus according to claim 14, wherein the first locking has a first locking portion, the second locking disc has a second locking portion, the first locking portion being locked to and engaged with the second locking portion, and
wherein the first locking portion and the second locking portion are tooth-shaped, or the first locking portion fits the second locking portion in a manner that a protrusion fits a groove.

16. The joint locking apparatus according to claim 14, wherein the lever assembly comprises a connecting shaft and a locking lever rotatably connected to the connecting shaft, the connecting shaft extending through the guide pressing plate and the guide body and is connected to the first locking disc and the second locking disc, the locking lever drives the guide pressing plate to abut against the guide body through the connecting shaft, and allows the first locking disc to engage with and lock to the second locking disc.

17. The joint locking apparatus according to claim 16, wherein an end portion of the locking lever connected to the connecting shaft has a cam shape.

18. The joint locking apparatus according to claim 17, wherein the lever assembly further comprises a stopper configured to sleeve over the connecting shaft and provided on the guide sleeve, the stopper being configured to limit a position of the locking lever when the locking lever is locked.

19. The joint locking apparatus according to claim 18, wherein the stopper is provided with a restricting gap, and the locking lever is located within the restricting gap when the locking lever is locked.

20. A head brace connection apparatus, comprising a plurality of joint locking apparatuses according to any one of claims 10 to 19, the plurality of joint locking apparatuses being connected in series,
wherein a first link of one of the joint locking apparatuses is connected to a first link or a second link of an adjacent joint locking apparatus, and/or, a second link of one of the joint locking apparatuses is connected to a second link or a first link of an adjacent joint locking apparatus.

21. A surgery assisting robot system, comprising a support frame, a surgical robotic arm arranged with the support frame, and a head brace connection apparatus according to claim 20,
wherein the surgical robotic arm is movably arranged with the support frame, and the head brace connection apparatus is arranged with the support frame and is movable with respect to the support frame, such that the head brace connection apparatus connects to and fixes the head brace apparatus.

22. A head brace fixing apparatus, provided in a surgery assisting robot system and connected to a head brace apparatus of a hospital bed system, comprising:
a telescoping assembly, telescopically arranged with a support frame of the surgery assisting robot system and connectable to the head brace apparatus; and
a second locking assembly, configured to lock or unlock the telescoping assembly, the second locking assembly limiting a movement of the telescoping assembly when locking the telescoping assembly.

23. The head brace fixing apparatus according to claim 22, wherein the telescoping assembly comprises a support arm and a linear motion member, the support arm being movable with respect to the support frame through the linear motion member.

24. The head brace fixing apparatus according to claim 23, wherein the linear motion member comprises a third guide member and a slide member slidingly engaged with the third guide member, the third guide member being provided in an axial direction of the support arm, the slide member being arranged with the support frame.

25. The head brace fixing apparatus according to claim 23, wherein the second locking assembly comprises a transmission member and a braking member, the braking member being arranged with the support frame, the braking member extending through the transmission member to connect to the support arm.

26. The head brace fixing apparatus according to claim 25, wherein the transmission member comprises a locking gear and a locking rack engaged with each other, the locking gear being rotatably connected to the braking member, the locking rack being provided in an axial direction of the support arm, and the locking gear locking the locking rack when the braking member locks the locking gear.

27. The head brace fixing apparatus according to claim 25, wherein the braking member comprises an electromagnetic brake with a friction plate, and the friction plate is in contact with the locking gear when the electromagnetic brake is powered off.

28. The head brace fixing apparatus according to claim 24, wherein the head brace fixing apparatus further comprises a restricting assembly provided on the support arm and configured to limit a movement of the linear motion member.

29. The head brace fixing apparatus according to claim 28, wherein the restricting assembly comprises a restricting member provided at an end portion of the support arm and configured to limit a motion stroke of the third guide member.

30. The head brace fixing apparatus according to claim 29, wherein the restricting assembly further comprises a shock absorber provided on a surface of the restricting member facing the third guide member, the shock absorber being capable of abutting against the slide member.

31. A surgery assisting robot system, comprising a support frame, a surgical robotic arm arranged with the support frame, and a head brace fixing apparatus according to any one of claims 22 to 30,
wherein the surgical robotic arm is movably arranged with the support frame, and the head brace fixing apparatus is arranged with the support frame and is telescopic with respect to the support frame, the head brace fixing apparatus, when extending out of the support frame, is connected to and fix the head brace apparatus.
